# EUROPÄISCHE PATENTSCHRIFT

(11) **EP 4 385 462 B1**
(45) Veröffentlichungstag und Bekanntmachung des Hinweises auf die Patenterteilung: **01.04.2026**
(21) Anmeldenummer: 23216655.3
(22) Anmeldetag: 14.12.2023
(51) Int. Cl.: A61F 2/34, A61B 17/16, A61F 2/36, A61F 2/46, A61F 2/30

(54) **MODULARES PROBEIMPLANTATSYSTEM**
MODULAR TRIAL IMPLANT SYSTEM
SYSTÈME D'IMPLANT D'ÉCHANTILLON MODULAIRE

(30) Priorität: 15.12.2022 DE 102022133453
(43) Veröffentlichungstag der Anmeldung: 19.06.2024
(73) Patentinhaber: Aesculap AG, 78532 Tuttlingen (DE)
(72) Erfinder: Saueressig, Thomas, 78532 Tuttlingen (DE)
(74) Vertreter: Hoeger, Stellrecht & Partner Patentanwälte mbB

(56) Entgegenhaltungen:
- DE-U1- 202008 008 565
- DE-U1- 202008 008 566
- US-A1- 2012 239 160
- US-A1- 2019 159 905

## Beschreibung

Die vorliegende Erfindung ist in Anspruch 1 definiert und betrifft ein modulares Probeimplantatsystem umfassend mindestens ein erstes Probeimplantatteil, welches ausgebildet ist zum lösbaren Verbinden mit mindestens einem zweiten Probeimplantatteil und/oder einem Implantatteil zur Ausbildung mindestens eines ersten Probegelenkimplantatteils, wobei das mindestens eine erste Probeimplantatteil eine Konusaufnahme zum Aufnehmen eines Verbindungskonus des mindestens einen zweiten Probeimplantatteils oder des mindestens einen Implantatteils umfasst, wobei die Konusaufnahme eine Implantatlängsachse definiert, wobei das mindestens eine erste Probeimplantatteil mindestens zwei einander zugeordnete erste Rastelemente und mindestens zwei einander zugeordnete zweite Rastelemente umfasst, wobei die mindestens zwei ersten Rastelemente einerseits und die mindestens zwei zweiten Rastelemente andererseits in der Konusaufnahme bezogen auf die Implantatlängsachse axial versetzt angeordnet oder ausgebildet sind zum Hintergreifen unterschiedlich langer Verbindungskonen zweiter Probeimplantatteile oder Implantatteile.

Ein Probeimplantatsystem der eingangs beschriebenen Art ist beispielsweise aus der EP 2 429 456 B1 oder der US 2012/0239160 A1 bekannt. Die in diesen Veröffentlichungen beschriebenen modularen Probeköpfe, welche erste Probeimplantatteile im Sinne des eingangs beschriebenen modularen Probeimplantatsystems bilden, werden insbesondere temporär mit Konen von Schäften von Hüftgelenkendoprothesen während einer Implantation derselben gekoppelt, um eine Probereposition durchzuführen. Bei den Schäften kann es sich beispielsweise um Probeimplantatteile handeln, die nur temporär während einer Operation in den Körper eines Patienten eingesetzt werden, oder aber auch um Implantatteile, die für den dauerhaften Verbleib im Körper eines Patienten vorgesehen und bereits in eine Knochenkavität des Patienten eingesetzt sind.

Ein Problem bei den bekannten Probeköpfen ist insbesondere, dass sich die als Finger bezeichneten Vorsprünge an den Probeköpfen beim Einführen derselben in einen Operationssitus zum Koppeln mit einem Konus - und auch wieder beim Entfernen derselben - Weichteile verklemmen und mitziehen oder anderweitig befördern können. Zudem ist auch eine Reinigung derart komplexer Geometrien, wenn sich Gewebe oder Flüssigkeiten in Zwischenräumen der Finger sammeln, nur mit einer manuellen Vorreinigung sicher zu erreichen.

In der DE 20 2008 008 566 U1 sowie in der DE 20 2008 008 565 U1 sind weitere modulare Probeimplantatsysteme beschrieben. Eine modulare Vorrichtung zum Einstellen eines Abstands zwischen einem Kopf- und einem Schaftelement eines Implantats ist aus der US 2019/0159905 A1 bekannt.

Es ist daher eine Aufgabe der vorliegenden Erfindung, ein modulares Probeimplantatsystem der eingangs beschriebenen Art zu verbessern, dass es insbesondere besser handhabbar ist.

Diese Aufgabe wird bei einem modularen Probeimplantatsystem der eingangs beschriebenen Art erfindungsgemäß dadurch gelöst, dass das mindestens eine erste Probeimplantatteil eine die Konusaufnahme definierende Aufnahmehülse umfasst, dass die Aufnahmehülse eine die Implantatlängsachse umgebende und die Konusaufnahme begrenzende Hülsenwand umfasst und dass die Hülsenwand mindestens in einem ersten Bereich zwischen den mindestens zwei ersten Rastelementen und mindestens in einem zweiten Bereich zwischen den mindestens zwei zweiten Rastelementen einschnitt- und/oder durchbrechungsfrei ausgebildet ist.

Ein derart weitergebildetes modulares Probeimplantatsystem ist im Vergleich zu dem aus der EP 2 429 456 B1 bekannten Probeimplantatsystem signifikant besser handhabbar. Insbesondere dadurch, dass in den ersten und zweiten Bereichen keine Einschnitte und/oder Durchbrechungen vorgesehen sind, kann es beim Koppeln mit und auch beim Entfernen des ersten Probeimplantatteils von einem Verbindungskonus eines zweiten, zum temporären Verbleib im Körper eines Patienten eingesetzten Probeimplantatteils oder eines für die dauerhafte Implantation bestimmten Implantatteils nicht zu den oben beschriebenen Problemen kommen, die sich bei einem aus der EP 2 429 456 B1 bekannten Probekopf ergeben. Ein Verklemmen und mit sich Ziehen von Weichteilen ist ohne Einschnitte und Durchbrechungen in den ersten und zweiten Bereichen nämlich nicht möglich. Zudem vereinfacht sich auch die Reinigung des ersten Probeimplantatteils, so dass es keiner manuellen Vorreinigung desselben bedarf. Eine maschinelle Reinigung mit anschließender Heißdampfsterilisation ermöglicht insbesondere die mehrfache Verwendung der ersten Probeimplantatteile des modularen Probeimplantatsystems. Insgesamt ergibt sich so eine verbesserte Handhabbarkeit des Probeimplantatsystems für einen Operateur. Zweite Probeimplantatteile können insbesondere auch durch Raspelkörper von Raspelwerkzeugen gebildet werden. Derartige Raspelkörper sind beispielsweise mit dafür vorgesehenen Griffen der Raspelwerkzeuge lösbar verbindbar.

Günstigerweise ist die Hülsenwand vollständig einschnitt- und/oder durch-brechungsfrei ausgebildet. Auf diese Weise kann die Handhabbarkeit des modularen Probeimplantatsystems insbesondere noch weiter verbessert werden. Insbesondere kann es so nicht zu einer unerwünschten Wechselwirkung mit Gewebe und Weichteilen eines Patienten beim Einsetzen und Entfernen des ersten Probeimplantatteils in den Körper eines Patienten zum Koppeln mit einem Verbindungskonus eines zweiten Probeimplantatteils kommen. Auch die Reinigbarkeit ist insbesondere wie bereits erläutert signifikant verbessert.

Um das Einführen des ersten Probeimplantatteils in einen Körper eines Patienten zu verbessern, ist es vorteilhaft, wenn die Konusaufnahme eine Einführöffnung zum Einführen des Verbindungskonus umfasst und wenn die Einführöffnung von einem kreisringförmigen, unterbrechungsfreien Einführöffnungsrand begrenzt ist. Ein solcher Einführöffnungsrand, welcher umlaufend ist, bietet keine Angriffspunkte zum Verhaken mit Gewebe und Weichteilen. So lässt sich ein erstes Probeimplantatteil von einem Operateur sicher mit einem Verbindungskonus eines zweiten Probeimplantatteils koppeln und auch wieder von diesem entfernen. Auch ist ein solcher umlaufender unterbrechungsfreier Einführöffnungsrand gut reinigbar.

Günstig ist es, wenn der Einführöffnungsrand eine Randebene definiert und wenn sich die Randebene quer, insbesondere senkrecht, zur Implantatlängsachse erstreckt. Ein derart ausgestalteter Einführöffnungsrand kann sich insbesondere mit Weichteilen und Gewebe eines Patienten praktisch nicht verhaken. So kann das erste Probeimplantatteil von einem Operateur einfach und sicher mit einem Verbindungskonus eines zweiten Probeimplantatteils oder eines Implantatteils gekoppelt werden.

Gemäß einer weiteren bevorzugten Ausführungsform kann vorgesehen sein, dass sich die Konusaufnahme in Richtung auf einen Konusboden hin konisch verjüngt und dass sich der Konusboden quer, insbesondere senkrecht, zur Implantatlängsachse erstreckt. Der Konusboden kann insbesondere als Anschlag für eine, beispielsweise ebene, Endfläche des Verbindungskonus dienen. Wird das erste Probeimplantatteil mit einer hinreichenden Geschwindigkeit mit dem Konusboden gegen eine Endfläche des Verbindungskonus bewegt, entsteht ein für einen Operateur hörbares Klickgeräusch. Dieses Geräusch signalisiert ihm, dass die Probeimplantatteile in gewünschter Weise miteinander gekoppelt sind. Der Konusboden kann insbesondere durchbrechungsfrei ausgebildet sein.

Vorteilhaft ist es, wenn am mindestens einen ersten Probeimplantatteil eine Durchbrechung ausgebildet ist und dass die Durchbrechung den Konusboden durchsetzt. Auf diese Weise kann insbesondere eine Entlüftung der Konusaufnahme realisiert werden, damit insbesondere eingeschlossene Luft aus der Konusaufnahme entweichen kann, die sonst beim Aufsetzen des mindestens einen ersten Probeimplantatteils auf den Verbindungskonus komprimiert werden müsste. Überdies würde eine solche Lösung, also ohne Durchbrechung, eine akustische Rückmeldung beim Aufschnappen und Verrasten des ersten Probeimplantatteils auf dem Verbindungskonus des zweiten Probeimplantatteils oder des Implantatteils praktisch unmöglich machen. Auch können Gewebe und Flüssigkeiten durch die Durchbrechung aus der Konusaufnahme entweichen, so dass insbesondere ein leichtes Zusammenführen und Aufschnappen des ersten Probeimplantatteils auf den Verbindungskonus des zweiten Probeimplantatteils ermöglicht wird.

Das modulare Probeimplantatsystem lässt sich insbesondere auf einfache Weise ausbilden, wenn die Durchbrechung koaxial zur Implantatlängsachse angeordnet oder ausgebildet ist.

Günstig ist es, wenn der Konusboden eine in Richtung auf die Einführöffnung hin weisende Anschlagfläche für eine Konusendfläche des Verbindungskonus bildet. Insbesondere kann die Anschlagfläche ringförmig ausgebildet sein. Eine ringförmige Anschlagfläche kann insbesondere auf einfache Weise realisiert werden, wenn der Konusboden eine kreisringförmige Durchbrechung aufweist. Die Anschlagfläche kann wie bereits erläutert einem Operateur eine akustische Rückmeldung geben, wenn die zusammenwirkenden ersten und zweiten Probeimplantatteile oder die zusammenwirkenden ersten Probeimplantatteile und Implantatteile in definierter Weise miteinander gekoppelt werden.

Vorzugsweise ist ein erster Abstand der mindestes zwei ersten Rastelemente vom Konusboden kleiner als ein zweiter Abstand der mindestens zwei zweiten Rastelemente vom Konusboden. Diese Ausgestaltung ermöglicht es insbesondere, das erste Probeimplantatteil mit unterschiedlich langen Verbindungskonen zweiter Probeimplantatteile zu koppeln. Eine Kopplung kann insbesondere durch ein Hintergreifen des Verbindungskonus erfolgen, und zwar in einem Bereich, in welchem sich an den sich erweiternden Verbindungskonus eine Einschnürung anschließt, wie sie bei Femurschäften von Hüftgelenkendoprothesen oder auch hierfür genutzten Raspelkörpern modularer Raspeln üblicherweise vorgesehen ist. Auf diese Weise lässt sich die Zahl der erforderlichen Komponenten des modularen Probeimplantatsystems halbieren, da jedes erste Probeimplantatteil mit zwei unterschiedlich langen Verbindungskonen gekoppelt werden kann. Es müssen also nicht für unterschiedlich lange Verbindungskonen unterschiedliche erste Probeimplantatteile, beispielsweise in Form von Probeköpfen, bereitgestellt werden. Die ersten Probeimplantatteile können wie erläutert mit zwei unterschiedlich langen Verbindungskonen in definierter Weise gekoppelt werden.

Um ein einfaches und sicheres Koppeln des ersten Probeimplantatteils mit einem Verbindungskonus eines zweiten Probeimplantatteils oder eines zweiten Implantatteils zu ermöglichen, ist es günstig, wenn sich die mindestens zwei ersten Rastelemente bezogen auf die Implantatlängsachse in Umfangsrichtung erstrecken. So können sie gleichzeitig einen Verbindungskonus hintergreifen.

Ferner ist es günstig, wenn die mindestens zwei ersten Rastelemente gleichmäßig über einen Umfang der Hülsenwand verteilt angeordnet oder ausgebildet sind. Sind beispielsweise nur zwei erste Rastelemente vorgesehen, können sich diese bezogen auf die Implantatlängsachse diametral gegenüberliegen. Es können aber auch drei oder vier erste Rastelemente vorgesehen sein, die dann um entsprechende Versatzwinkel, nämlich entweder 120° oder 90°, versetzt über den Umfang der Hülsenwand verteilt angeordnet oder ausgebildet werden.

In entsprechender Weise ist es vorteilhaft, wenn sich die mindestens zwei zweiten Rastelemente bezogen auf die Implantatlängsachse in Umfangsrichtung erstrecken. Auch sie können so einen Verbindungskonus an einem zweiten Probeimplantatteil oder einem Implantatteil gleichzeitig hintergreifen.

Vorzugsweise sind die mindestens zwei zweiten Rastelemente gleichmäßig über einen Umfang der Hülsenwand verteilt angeordnet oder ausgebildet. Es können beispielsweise zwei, drei, vier oder mehr zweite Rastelemente vorgesehen sein. Diese lassen sich dann gleichmäßig mit einem Verbindungskonus verrasten, indem sie diesen beim Aufsetzen hintergreifen.

Für ein einfaches und zuverlässiges Koppeln des ersten Probeimplantatteils mit einem Verbindungskonus eines zweiten Probeimplantatteils oder eines Implantatteils ist es günstig, wenn die mindestens zwei ersten Rastelemente eine erste Rastelementebene definieren und wenn sich die erste Rastelementebene quer, insbesondere senkrecht, zur Implantatlängsachse erstreckt.

In ähnlicher Weise ist es vorteilhaft, wenn die mindestens zwei zweiten Rastelemente eine zweite Rastelementebene definieren und wenn sich die zweite Rastelementebene quer, insbesondere senkrecht, zur Implantatlängsachse erstreckt.

Auf einfache Weise lässt sich das modulare Probeimplantatsystem ausbilden, wenn die mindestens zwei ersten Rastelemente in Form von in Richtung auf die Implantatlängsachse hin weisenden ersten Rastvorsprüngen ausgebildet sind. Diese können insbesondere eine Kante in einem Übergangsbereich des Verbindungskonus zu einem sich an diesen anschließenden Halsbereich mit verringertem Querschnitt sicher hintergreifen und dort einrasten.

In ebensolcher Weise ist es vorteilhaft, wenn die mindestens zwei zweiten Rastelemente in Form von in Richtung auf die Implantatlängsachse hin weisenden zweiten Rastvorsprüngen ausgebildet sind.

Gemäß einer weiteren bevorzugten Ausführungsform kann vorgesehen sein, dass die mindestens zwei ersten Rastelemente und die mindestens zwei zweiten Rastelemente in Umfangsrichtung versetzt zueinander angeordnet oder ausgebildet sind derart, dass in Umfangsrichtung jedes erste Rastelement zwischen zwei zweiten Rastelementen und jedes zweite Rastelement zwischen zwei ersten Rastelementen angeordnet oder ausgebildet ist. Zu beachten ist insbesondere, dass die ersten und zweiten Rastelemente in unterschiedlichen Rastelementebenen liegen, da sie relativ zueinander bezogen auf die Implantatlängsachse axial versetzt angeordnet beziehungsweise ausgebildet sind. Die wie vorgeschlagen versetzte Anordnung der ersten und zweiten Rastelemente in Umfangsrichtung ermöglicht es insbesondere, dass eine unerwünschte, jedoch nicht vollständig vermeidbare Wechselwirkung der ersten Rastelemente beim Koppeln des ersten Probeimplantatteils mit einem längeren Verbindungskonus, welcher mit den zweiten Rastelementen zusammenwirkend verrastet, minimiert werden kann. Insbesondere kann so sowohl beim Aufschnappen des ersten Probeimplantatteils auf einen kürzeren Verbindungskonus als auch beim Aufschnappen auf einen längeren Verbindungskonus ein Rast-, Schnapp- oder Klickgeräusch erzeugt werden, das einem Operateur als akustische Rückmeldung für eine bestimmungsgemäße und tatsächlich erfolgte Kopplung der beiden Probeimplantatteile miteinander oder des ersten Probeimplantatteils und eines Implantatteils miteinander dienen kann.

Um die Herstellung des modularen Probeimplantatsystems zu vereinfachen, ist es vorteilhaft, wenn zwei erste Rastelemente vorgesehen sind und wenn die zwei ersten Rastelemente bezogen auf die Implantatlängsachse einander diametral gegenüberliegend angeordnet oder ausgebildet sind. Dies ist so zu verstehen, dass nur zwei erste Rastelemente vorgesehen sind. Zwei erste Rastelemente sind dem Grunde nach ausreichend, um eine optimale Rast-/ Schnappverbindung mit einem Verbindungskonus eines zweiten Probeimplantatteils oder eines Implantatteils einzugehen.

Ferner ist es günstig, wenn zwei zweite Rastelemente vorgesehen sind und wenn die zwei zweiten Rastelemente bezogen auf die Implantatlängsachse einander diametral gegenüberliegend angeordnet oder ausgebildet sind. Auch diese Ausgestaltung ermöglicht ein definiertes und sicheres Verrasten des ersten Probeimplantatteils mit einem Verbindungskonus eines zweiten Probeimplantatteils oder eines Implantatteils.

Gemäß einer weiteren bevorzugten Ausführungsform kann vorgesehen sein, dass sich die mindestens zwei ersten Rastelemente über einen ersten Umfangswinkel bezogen auf die Implantatlängsachse erstrecken, dass sich die mindestens zwei zweiten Rastelemente über einen zweiten Umfangswinkel bezogen auf die Implantatlängsachse erstrecken und dass der erste Umfangswinkel größer ist als der zweite Umfangswinkel. Durch die geringere Erstreckung der zweiten Rastelemente in Umfangsrichtung ergibt sich eine verbesserte Verformbarkeit der Hülsenwand im Bereich derselben. Dies hat insbesondere den Vorteil, dass die ersten Rastelemente beim Einführen eines längeren Verbindungskonus diesen nicht vollständig abbremsen können, so dass es trotzdem zu einem Schnappeffekt und einem hörbaren Anschlagen kommen kann beim Zusammenwirken der zweiten Rastelemente mit einem längeren Verbindungskonus. Ein solcher Schnappeffekt beziehungsweise das entstehende Klickgeräusch werden durch einen schnellen ruckartigen Kontakt der Konusendfläche des Verbindungskonus mit dem Konusboden bewirkt. Sobald die Rastelemente über einen Rand des Verbindungskonus geschoben werden, kann das erste Probeimplantatteil beschleunigt werden, so dass es mit einer zunehmenden Geschwindigkeit mit dem Konusboden auf die Konusendfläche auftreffen und dadurch ein Klickgeräusch hervorrufen kann.

Günstig ist es, wenn der erste Umfangswinkel einen Wert in einem Bereich von etwa 30° bis etwa 50° aufweist, insbesondere in einem Bereich von etwa 35° bis etwa 45°, und wenn der zweite Umfangswinkel einen Wert in einem Bereich von etwa 20° bis etwa 30° aufweist, insbesondere in einem Bereich von etwa 22° bis etwa 28°. Vorzugsweise beträgt der erste Umfangswinkel etwa 40° pro Rastelement und der zweite Umfangswinkel etwa 25° pro Rastelement. Insbesondere können diese Werte vorgesehen sein, wenn nur jeweils zwei erste und zweite Rastelemente vorgesehen sind. Den Umfangswinkel in den angegebenen Bereichen vorzusehen, stellt insbesondere die gewünschte Funktionalität des modularen Probeimplantatsystems sicher.

Günstig ist es, wenn die Konusaufnahme eine Innenwandfläche definiert, wenn an der Innenwandfläche ein erster, bezogen auf die Implantatlängsachse hohlzylindrischer Abschnitt ausgebildet ist und wenn die mindestens zwei ersten Rastelemente am ersten hohlzylindrischen Abschnitt ausgebildet sind. Diese Weiterbildung ermöglicht es insbesondere, dass ein Zusammenwirken der ersten Rastelemente mit einem zugeordneten kürzeren Verbindungskonus in optimaler Weise ermöglicht wird. Insbesondere kann so ein Kontakt des Verbindungskonus mit der Konusaufnahme im Bereich der ersten Rastelemente eben genau auf diese beschränkt werden. Ein Kontakt mit dem hohlzylindrischen Abschnitt kann durch diese Ausgestaltung vermieden werden.

Vorteilhaft ist es, wenn an der Innenwandfläche ein zweiter, bezogen auf die Implantatlängsachse hohlzylindrischer Abschnitt ausgebildet ist und wenn die mindestens zwei zweiten Rastelemente am zweiten hohlzylindrischen Abschnitt ausgebildet sind. Auch bei den zweiten Rastelementen ergibt sich wie bei den ersten Rastelementen in Verbindung mit dem ersten hohlzylindrischen Abschnitt der Vorteil, dass hier nur ein Zusammenwirken mit dem Verbindungskonus mit den zweiten Rastelementen, nicht jedoch mit der Innenfläche der Konusaufnahme im Bereich des zweiten hohlzylindrischen Abschnitts ermöglicht wird.

Gemäß einer weiteren bevorzugten Ausführungsform kann vorgesehen sein, dass an der Konusaufnahme eine bezogen auf die Implantatlängsachse umlaufende Nut angeordnet oder ausgebildet ist und dass die Nut zwischen den mindestens zwei ersten Rastelementen einerseits und den mindestens zwei zweiten Rastelementen andererseits angeordnet oder ausgebildet ist. Die Nut bildet eine Schwächung der Hülsenwand, wodurch diese im Bereich der Nut flexibler beziehungsweise elastischer wird. Dies ist vorteilhaft für die Funktion der zweiten Rastelemente. So kann insbesondere sichergestellt werden, dass auch das Verrasten der zweiten Rastelemente mit einem längeren Verbindungskonus zu einer beschleunigten Bewegung des ersten Probeimplantatteils beim Aufschnappen auf den Verbindungskonus führt, um das für einen Operateur gewünschte Klickgeräusch zu erzeugen.

Auf einfache Weise lässt sich das modulare Probeimplantatsystem ausbilden, wenn die Nut in Richtung auf die Implantatlängsachse hin weisend konkav gekrümmt ist. Beispielsweise kann sie mit einem kleinen Radius ausgebildet sein, welcher einen Wert in einem Bereich von etwa 1 mm bis etwa 2 mm aufweist.

Vorteilhaft ist es, wenn die Nut eine Nutebene definiert und wenn die Nutebene quer, insbesondere senkrecht, zur Implantatlängsachse verläuft. Beispielsweise kann die Nutebene so parallel zu den ersten und zweiten Rastelementebenen verlaufen, insbesondere zwischen diesen.

Vorzugsweise erstreckt sich die Nut zwischen dem ersten hohlzylindrischen Abschnitt und dem zweiten hohlzylindrischen Abschnitt. Es ist nicht zwingend erforderlich, dass die Nut direkt an die beiden hohlzylindrischen Abschnitte angrenzt.

Günstigerweise grenzt die Nut direkt an den ersten hohlzylindrischen Abschnitt und/oder direkt an den zweiten hohlzylindrischen Abschnitt an. So kann sie insbesondere bei einem direkten Angrenzen an den zweiten hohlzylindrischen Abschnitt eine optimale Wirkung der mindestens zwei zweiten Rastelemente in der beschriebenen Weise ermöglichen.

Gemäß einer weiteren bevorzugten Ausführungsform kann vorgesehen sein, dass die Hülsenwand einen ersten Hülsenabschnitt und einen sich daran anschließenden zweiten Hülsenabschnitt umfasst, dass sich die Konusaufnahme vom zweiten Hülsenabschnitt in Richtung auf den ersten Hülsenabschnitt verjüngt, dass die mindestens zwei ersten und zweiten Rastelemente ausschließlich am zweiten Hülsenabschnitt angeordnet oder ausgebildet sind und dass eine erste Wanddicke des ersten Hülsenabschnitts größer ist als eine zweite Wanddicke des zweiten Hülsenabschnitts. Insbesondere ist die ersten Wanddicke mindestens etwa 50% größer als die zweite Wanddicke. Zum Beispiel kann sie etwa doppelt so groß sein wie die zweite Wanddicke. Den zweiten Hülsenabschnitt mit einer reduzierten Wanddicke oder Wandstärke auszubilden hat insbesondere den Vorteil, dass sich die Hülsenwand beim Aufschieben des ersten Probeimplantatteils auf einen Verbindungskonus eines zweiten Probeimplantatteils oder eines Implantatteils im Bereich des zweiten Hülsenabschnitts in der erforderlichen Weise leicht verformen kann. Der erste Hülsenabschnitt dagegen ist mit der ersten Wanddicke stabiler und weniger elastisch ausgebildet. So kann die Funktion des ersten Probeimplantatteils zum Rasten und Verbinden mit einem Verbindungskonus eines zweiten Implantatteils oder eines Implantatteils in definierter Weise vorgegeben und dadurch verbessert werden.

Günstigerweise ist das mindestens eine erste Probeimplantatteil aus einem Kunststoff ausgebildet ist. Insbesondere kann hier ein Kunststoff zum Einsatz kommen, der die gewünschte Elastizität, insbesondere im Bereich des zweiten Hülsenabschnitts beziehungsweise im Bereich der ersten und zweiten Rastelemente, aufweist. Vorzugsweise handelt es sich bei dem Kunststoff um einen heißdampfsterilisierbaren Kunststoff.

Günstig ist es, wenn der Kunststoff Polyphenylensulfon (PPSU) ist oder enthält. Polyphenylensulfon ist ein amorpher Werkstoff mit einer hohen Glasübergangstemperatur und einer niedrigen Feuchteaufnahme. Er eignet sich somit für hochwertige technische Teile und hoch beanspruchte Massenprodukte. Mit anderen Worten ist ein solcher Kunststoff zur Ausbildung von Probeimplantatteilen bestens geeignet.

Um eine Röntgenkontrolle des modularen Probeimplantatsystems zu ermöglichen, ist es vorteilhaft, wenn das mindestens eine erste Probeimplantatteil ein Röntgenkontrastmittel enthält.

Eine gute Sichtbarkeit des Probeimplantatsystems unter Röntgenkontrolle lässt sich erreichen, wenn das Röntgenkontrastmittel Bariumsulfat ist oder enthält.

Gemäß einer weiteren bevorzugten Ausführungsform kann vorgesehen sein, dass das modulare Probeimplantatsystem mindestens ein zweites Probeimplantatteil oder mindestens ein Implantatteil umfasst und dass das mindestens eine zweite Probeimplantatteil oder das mindestens eine Implantatteil einen in die Konusaufnahme einführbaren Verbindungskonus umfasst. So können die gekoppelten ersten und zweiten Probeimplantatteile beispielsweise einen ersten Probegelenkimplantatteil eines künstlichen Probegelenks bilden.

Vorzugsweise umfasst das modulare Probeimplantatsystem mindestens zwei zweite Probeimplantatteile oder mindestens zwei Implantatteile mit unterschiedlich langen Verbindungskonen. Diese können dann wie beschrieben mit dem ersten Probeimplantatteil mit seinen ersten beziehungsweise zweiten Rastelementen in Eingriff gebracht und gekoppelt werden.

Vorteilhaft ist es, wenn das mindestens eine zweite Probeimplantatteil in Form eines in eine Knochenkavität einsetzbaren Probeschafts oder in Form eines Raspelkörpers ausgebildet ist. Beispielsweise kann mit einem Raspelkörper eine Knochenkavität präpariert werden. Ist der Raspelkörper in die Knochenkavität eingesetzt, kann er zum Bestimmen einer Größe der endgültig zu implantierenden Implantatkomponenten mit einem in Form eines Probekopfs ausgebildeten oder einen solchen umfassenden ersten Probeimplantatteils in der oben beschriebenen Weise rastend gekoppelt werden.

Günstigerweise weist das mindestens eine erste Probeimplantatteil einen kugeligen oder kugelförmigen Probegelenkkopf auf. Ein solcher Probegelenkkopf kann beispielsweise mit einer Probegelenkpfanne eines künstlichen Probehüftgelenks zusammenwirken, um die optimale Größe der dauerhaft zu implantierenden Implantatkomponenten zu bestimmen.

Auf einfache Weise lässt sich das erste Probeimplantatteil ausbilden, wenn der Probegelenkkopf einen bezogen auf die Implantatlängsachse rotationssymmetrischen Ausschnitt einer Kugeloberfläche aufweist.

Günstig ist es, wenn die Durchbrechung die Konusaufnahme und die Kugeloberfläche fluidwirksam verbindet. So lässt sich die Konusaufnahme wie erläutert optimal entlüften. Zudem kann durch die Durchbrechung auch Gewebe und Flüssigkeit aus der Konusaufnahme sicher entweichen.

Vorteilhaft ist es, wenn das modulare Probeimplantatsystem mindestens ein künstliches Probegelenk mit einem ersten Probegelenkimplantatteil und einem mit diesem gelenkig zusammenwirkenden zweiten Probegelenkimplantatteil umfasst. Mit einem solchen Probegelenk können bei einem Patienten Größe und Form der endgültig und dauerhaft zu implantierenden Implantatkomponenten zuverlässig bestimmt werden.

Vorteilhaft ist es, wenn das künstliche Probegelenk in Form eines Probehüftgelenks ausgebildet ist, wenn das erste Probegelenkimplantatteil einen Prothesenschaft mit Probegelenkkopf bildet und wenn das zweite Probegelenkimplantatteil in Form einer Probegelenkpfanne ausgebildet ist. Auf diese Weise können mit dem modularen Probeimplantatsystem von einem Operateur geeignete Implantatkomponenten sowohl hinsichtlich ihrer Form also auch ihrer Größe für dauerhaft zu implantierende Hüftgelenkendoprothesen zuverlässig ermittelt werden.

Günstig ist es, wenn das mindestens eine erste Probeimplantatteil und/oder das mindestens eine zweite Probeimplantatteil oder das Implantatteil einstückig, insbesondere monolithisch, ausgebildet sind. So lässt sich eine hohe Stabilität der Probeimplantatteile erreichen. Zudem kann so auch eine Gefahr minimiert werden, dass kleine Teile von den jeweiligen Probeimplantatteilen abfallen und beispielsweise in einem Operationssitus in unerwünschter Weise verloren gehen können.

Die nachfolgende Beschreibung bevorzugter Ausführungsformen der Erfindung dient im Zusammenhang mit den Zeichnungen der näheren Erläuterung. Es zeigen:
- Figur 1:: eine erste perspektivische Ansicht eines ersten Ausführungsbeispiels eines ersten Probeimplantatteils in Form eines Probekopfes mit Konusaufnahme;
- Figur 2:: eine weitere, perspektivische Ansicht des ersten Probeimplantatteils aus Figur 1 in teilweise durchbrochener Darstellung;

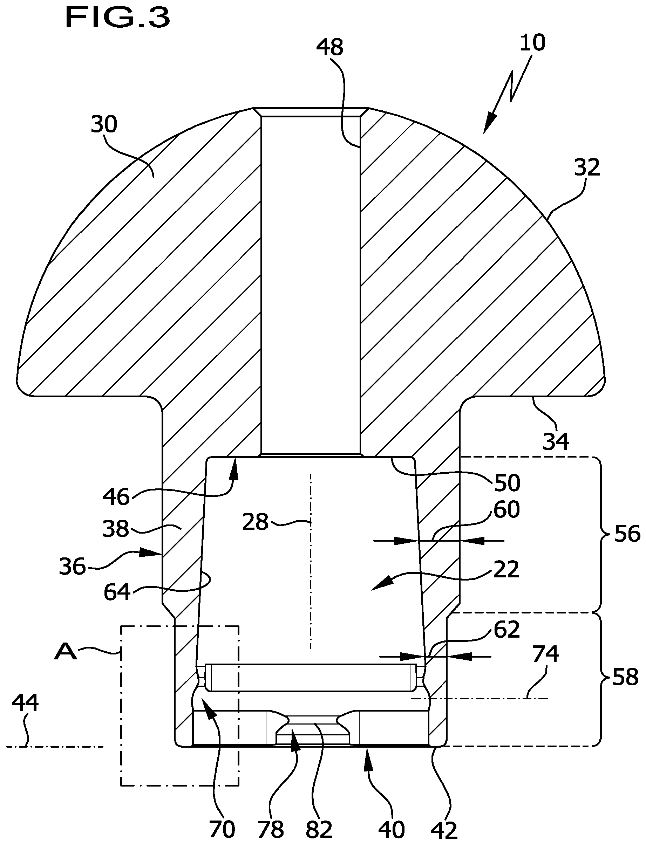

- Figur 3:: eine Schnittansicht längs Linie 3-3 in Figur 2;

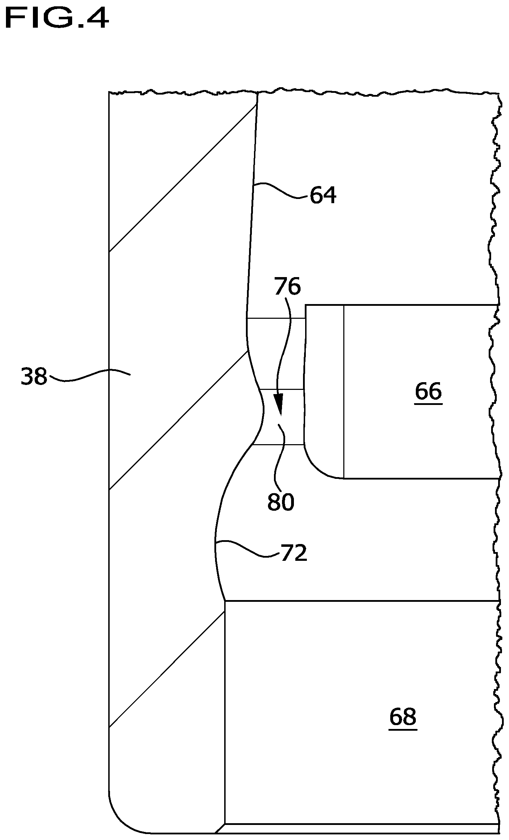

- Figur 4:: eine vergrößerte Ansicht des Bereichs A aus Figur 3;

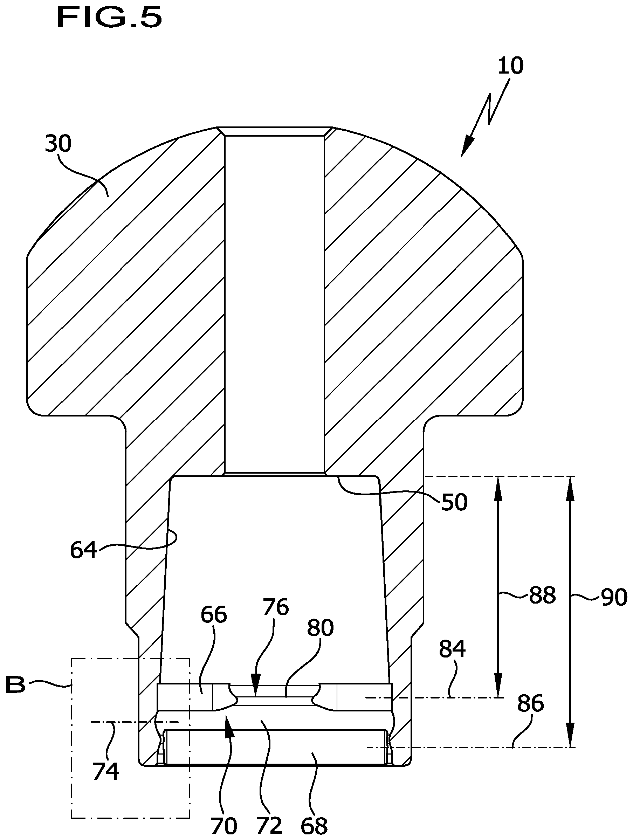

- Figur 5:: eine Schnittansicht längs Linie 5-5 in Figur 2;

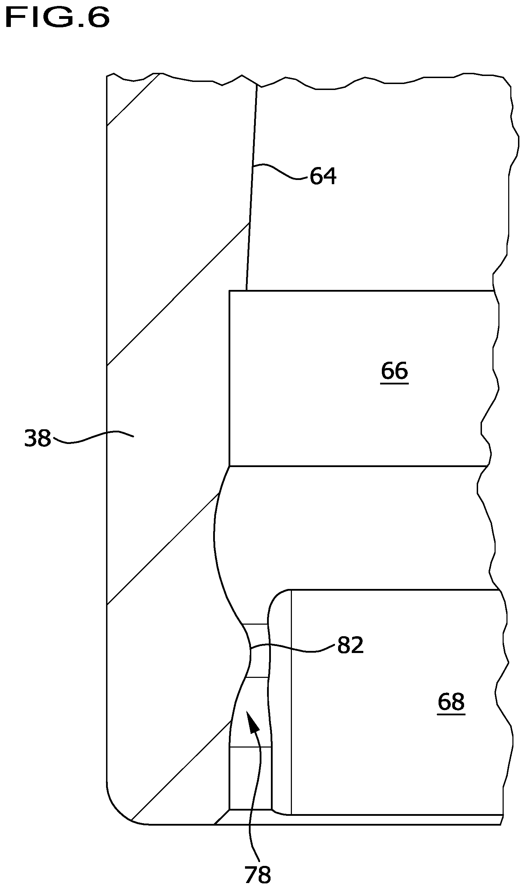

- Figur 6:: eine vergrößerte Teilansicht des Bereichs B aus Figur 5;
- Figur 7:: eine teilweise geschnittene Ansicht des Probekopfs aus Figur 1 mit einem ersten Ausführungsbeispiel eines zweiten Probeimplantatteils mit kurzem Verbindungskonus;
- Figur 8:: eine vergrößerte Teilansicht des Bereichs C in Figur 7;
- Figur 9:: eine teilweise geschnittene Ansicht des Probekopfs aus Figur 1 im Zusammenwirken mit einem zweiten Ausführungsbeispiel eines zweiten Probeimplantatteils mit einem längeren Verbindungskonus;

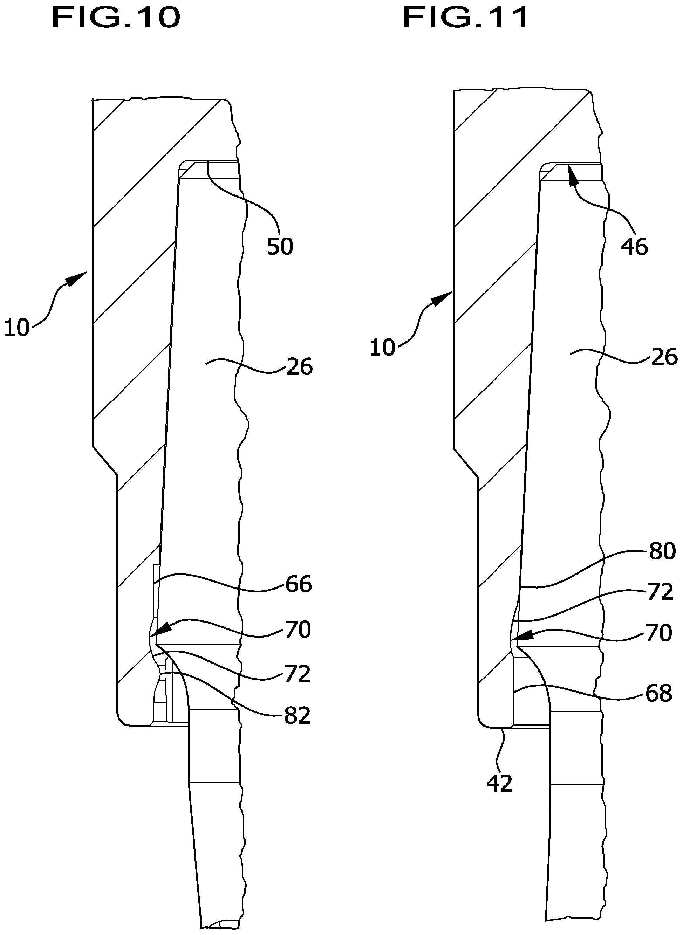

- Figur 10:: eine vergrößerte Teilansicht des Bereichs D aus Figur 9; und
- Figur 11:: eine Schnittansicht längs Linie 11-11 in Figur 9.

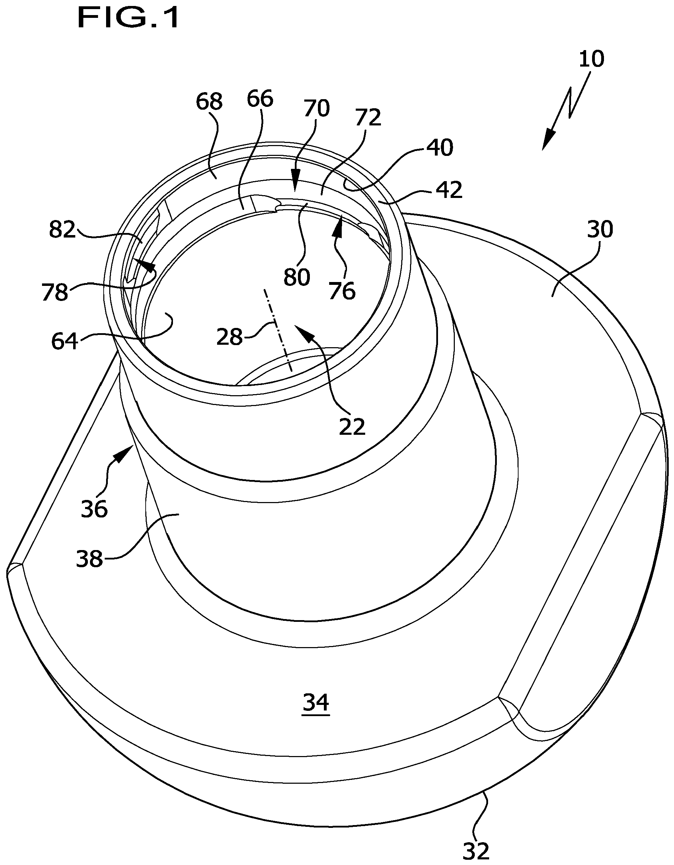

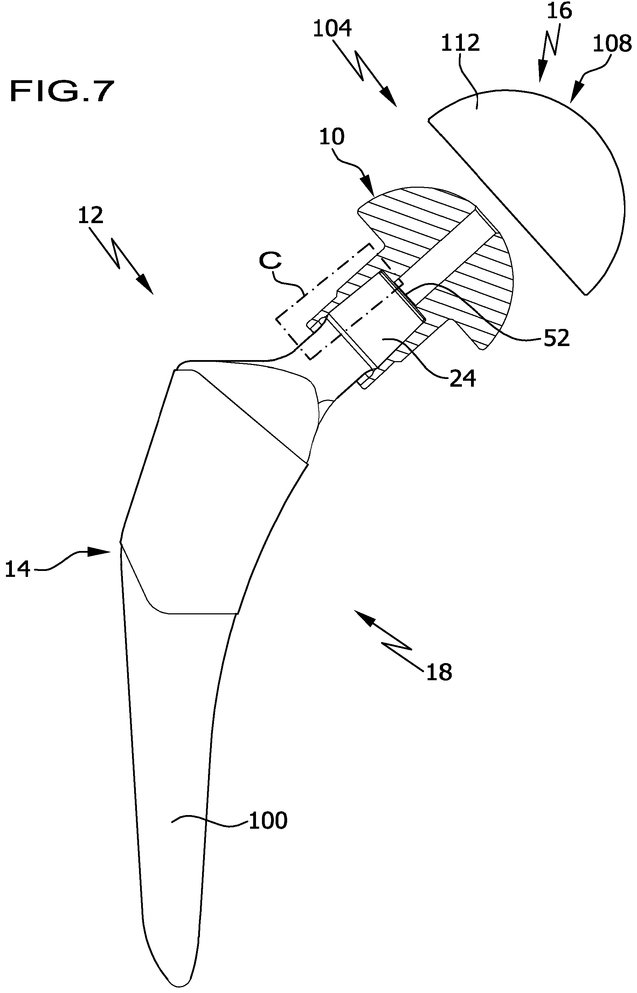

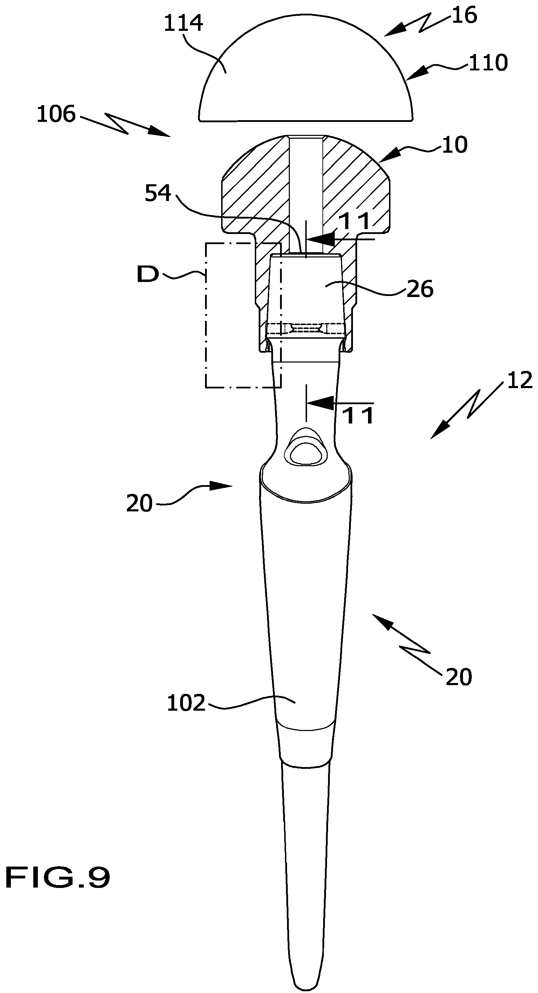

In Figur 1 ein erstes Ausführungsbeispiel eines ersten Probeimplantatteils 10 schematisch dargestellt. Es bildet einen Teil eines schematisch in den Figuren 7 und 9 dargestellten modularen Probeimplantatsystems 12.

Das erste Probeimplantatteil 10 ist ausgebildet zum lösbaren Verbinden mit zweiten Probeimplantatteilen 14 beziehungsweise 16, welche schematisch in den Figuren 7 und 9 dargestellt sind. Die miteinander verbundenen ersten und zweiten Probeimplantatteile 10 und 14 bilden ein erstes Probegelenkimplantatteil 18. Die miteinander gekoppelten ersten und zweiten Probeimplantatteile 10 und 16 bilden ein weiteres erstes Probegelenkimplantatteil 20.

Das erste Probeimplantatteil 10 umfasst eine Konusaufnahme 22 zum Aufnehmen eines Verbindungskonus 24 beziehungsweise 26 des zweiten Probeimplantatteils 14 beziehungsweise 16.

Die Konusaufnahme 22 definiert eine Implantatlängsachse 28.

Das erste Probeimplantatteil 10 umfasst einen Probegelenkkopf 30. Dieser ist kugelig beziehungsweise kugelförmig ausgebildet und definiert insgesamt eine Halbkugel. Seitlich ist die Halbkugel mit zwei parallel zueinander verlaufenden Abflachungen versehen, die eine manuelle Handhabung des ersten Probeimplantatteils erleichtern.

Der Probegelenkkopf 30 definiert einen Ausschnitt einer Kugeloberfläche 32. Dieser Ausschnitt ist bezogen auf die Implantatlängsachse 28 rotationssymmetrisch ausgebildet.

Von einer ebenen Unterseite 34 des Probegelenkkopfs 30 erstreckt sich koaxial zur Implantatlängsachse 28 eine Aufnahmehülse 36 weg. Die Aufnahmehülse 36 definiert die Konusaufnahme 22. Sie umfasst ferner eine die Implantatlängsachse 28 umgebende und die Konusaufnahme 22 begrenzende Hülsenwand 38.

Die Konusaufnahme 22 umfasst eine Einführöffnung 40 zum Einführen der Verbindungskonen 24, 26. Sie ist von einem kreisringförmigen, unterbrechungsfreien Einführöffnungsrand 42 begrenzt. Der Einführöffnungsrand 42 definiert eine Randebene 44, welche sich quer, bei dem in den Figuren dargestellten Ausführungsbeispiel senkrecht, zur Implantatlängsachse 28 erstreckt.

Die Konusaufnahme 22 verjüngt sich in Richtung auf einen Konusboden 46 hin konisch. Der Konusboden 46 erstreckt sich quer, bei dem in den Figuren dargestellten Ausführungsbeispiel senkrecht, zur Implantatlängsachse 28.

Am ersten Probeimplantatteil 10 ist ferner eine Durchbrechung 48 ausgebildet, welche die Konusaufnahme 22 und die Kugeloberfläche 32 fluidwirksam verbindet. Die Durchbrechung 48 durchsetzt den Konusboden 46. Sie ist koaxial zur Implantatlängsachse 28 ausgebildet.

Der Konusboden 46 bildet eine in Richtung auf die Einführöffnung 40 hin weisende Anschlagfläche 50 für Konusendflächen 52 beziehungsweise 54 der Verbindungskonen 24 beziehungsweise 26. Aufgrund der den Konusboden 46 koaxial zur Implantatlängsachse 28 durchsetzenden Durchbrechung 48 ist die Anschlagfläche 50 bei dem in den Figuren dargestellten Ausführungsbeispiel des ersten Probeimplantatteils 10 ringförmig ausgebildet, nämlich kreisringförmig.

Die Hülsenwand 38 umfasst einen ersten Hülsenabschnitt 56 und einen zweiten Hülsenabschnitt 58. Der erste Hülsenabschnitt 56 erstreckt sich ausgehend vom Konusboden 46 in Richtung auf den zweiten Hülsenabschnitt 58, welcher sich direkt an den ersten Hülsenabschnitt 56 anschließt. Auf diese Weise begrenzen die Hülsenabschnitte 56 und 58 die Konusaufnahme 22 derart, dass sich die Konusaufnahme 22 vom zweiten Hülsenabschnitt 58 in Richtung auf den ersten Hülsenabschnitt 56, und zwar bis zum Konusboden 46 hin, verjüngt.

Der erste Hülsenabschnitt 56 definiert eine erste Wanddicke 60. Der zweite Hülsenabschnitt 58 definiert eine zweite Wanddicke 62. Die erste Wanddicke 60 ist größer als die zweite Wanddicke 62, und zwar mindestens etwa 50% größer. Bei dem in den Figuren dargestellten Ausführungsbeispiel ist die erste Wanddicke 60 etwa doppelt so groß wie die zweite Wanddicke 62.

Die Konusaufnahme 22 definiert eine Innenwandfläche 64. Diese ist ausgehend vom Einführöffnungsrand 42 konisch geformt und verjüngt sich ausgehend vom Einführöffnungsrand 42 bis zum Konusboden 46 hin kontinuierlich.

Von der grundsätzlich konischen Innenwandfläche 64 abweichend sind an dieser ein erster hohlzylindrischer Abschnitt 66 und zweiter hohlzylindrischer Abschnitt 68 ausgebildet. Die Abschnitte 66 und 68 sind jeweils bezogen auf die Implantatlängsachse 28 hohlzylindrisch ausgebildet. Dies bedeutet, dass die Innenwandfläche 64 in den Bereichen der ersten und zweiten hohlzylindrischen Abschnitte 66 und 68 ihren jeweiligen Innendurchmesser beibehält und nicht verringert, wie dies im übrigen Bereich der Innenwandfläche 64 ausgehend vom Einführöffnungsrand 42 in Richtung auf den Konusboden 46 hin der Fall ist.

Der zweite hohlzylindrische Abschnitt 68 schließt unmittelbar an den Einführöffnungsrand 42 an.

Zwischen den ersten und zweiten hohlzylindrischen Abschnitten 66 und 68 ist am zweiten Hülsenabschnitt 58 eine Vertiefung 70 in Form einer Nut 72 ausgebildet. Die Nut 72 grenzt jeweils direkt an die hohlzylindrischen Abschnitte 66 und 68 an. Die Vertiefung 70 und damit auch die Nut 72 sind an der Konusaufnahme 22 bezogen auf die Implantatlängsachse 28 umlaufend angeordnet beziehungsweise ausgebildet. Die Nut 72 ist zudem in Richtung auf die Implantatlängsachse 28 hin weisend konkav gekrümmt. Überdies definiert die Nut 72 eine Nutebene 74, welche quer, bei dem in den Figuren dargestellten Ausführungsbeispiel senkrecht, zur Implantatlängsachse 28 verläuft.

Zum rastenden Verbinden des ersten Probeimplantatteils 10 mit einem der Verbindungskonen 24, 26 umfasst das erste Probeimplantatteil 10 zwei oder mehr einander zugeordnete erste Rastelemente 76 und zwei oder mehr einander zugeordnete zweite Rastelemente 78. Bei dem in den Figuren dargestellten Ausführungsbeispiel des ersten Probeimplantatteils 10 sind jeweils zwei erste Rastelemente 76 und zwei zweite Rastelemente 78 vorgesehen.

Die ersten und zweiten Rastelemente 76 und 78 sind ausschließlich am zweiten Hülsenabschnitt 58 angeordnet beziehungsweise ausgebildet.

Die zwei ersten Rastelemente 76 sind am ersten hohlzylindrischen Abschnitt 66 ausgebildet. Die zwei zweiten Rastelemente 78 sind dagegen am zweiten hohlzylindrischen Abschnitt 68 ausgebildet.

Die zwei ersten Rastelemente 76 sind gleichmäßig über einen Umfang der Hülsenwand 38, als insbesondere des ersten hohlzylindrischen Abschnitts 66, verteilt angeordnet beziehungsweise ausgebildet.

Die zwei zweiten Rastelemente 78 sind ebenfalls gleichmäßig über einen Umfang der Hülsenwand 38 verteilt angeordnet beziehungsweise ausgebildet, mithin also über einen Umfang des zweiten hohlzylindrischen Abschnitts 68.

Die zwei ersten Rastelemente 76 erstrecken sich bezogen auf die Implantatlängsachse 28 in Umfangsrichtung. Auch die zwei zweiten Rastelemente 78 erstrecken sich bezogen auf die Implantatlängsachse 28 in Umfangsrichtung.

Die zwei ersten Rastelemente 76 sind in Form von in Richtung auf die Implantatlängsachse 28 hin weisenden ersten Rastvorsprüngen 80 ausgebildet. Die zwei zweiten Rastelemente 78 sind ebenfalls in Form von in Richtung auf die Implantatlängsachse 28 hin weisenden zweiten Rastvorsprüngen 82 ausgebildet.

Die gleichmäßige Verteilung der ersten und zweiten Rastelemente 76 und 78 über einen Umfang der Hülsenwand 38 führt dazu, dass die zwei ersten Rastelemente 76 bezogen auf die Implantatlängsachse 28 einander diametral gegenüberliegend angeordnet beziehungsweise ausgebildet sind. Auch die zwei zweiten Rastelemente 78 sind bezogen auf die Implantatlängsachse 28 einander diametral gegenüberliegend angeordnet beziehungsweise ausgebildet.

Ferner definieren die ersten Rastelemente 76 eine erste Rastelementebene 84, welche sich quer, bei dem in den Figuren dargestellten Ausführungsbeispiel senkrecht, zur Implantatlängsachse 28 erstreckt. In ähnlicher Weise definieren die zweiten Rastelemente 78 eine zweite Rastelementebene 86, welche sich quer, bei dem in den Figuren dargestellten Ausführungsbeispiel senkrecht, zur Implantatlängsachse 28 erstreckt. Mithin verlaufen also die ersten und zweiten Rastelementebenen 84, 86 parallel zueinander sowie parallel zum Konusboden 46 und zur Randebene 44.

Die ersten und zweiten Rastelemente 76 und 78 sind an der Konusaufnahme 22 bezogen auf die Implantatlängsachse 28 somit axial versetzt zueinander angeordnet beziehungsweise ausgebildet. Ein erster Abstand 88 der ersten Rastelemente 76 vom Konusboden 46 ist somit kleiner als ein zweiter Abstand 90 der zweiten Rastelemente 78 vom Konusboden 46. Durch die unterschiedlichen Abstände 88 und 90 können die ersten und zweiten Rastelemente 76 und 78 die unterschiedlich langen Verbindungskonen 24, 26 in einer Kopplungsstellung, wie sie schematisch in den Figuren 7 und 9 für den einerseits kürzeren Verbindungskonus 24 und andererseits den längeren Verbindungskonus 26 schematisch dargestellt sind, hintergreifen.

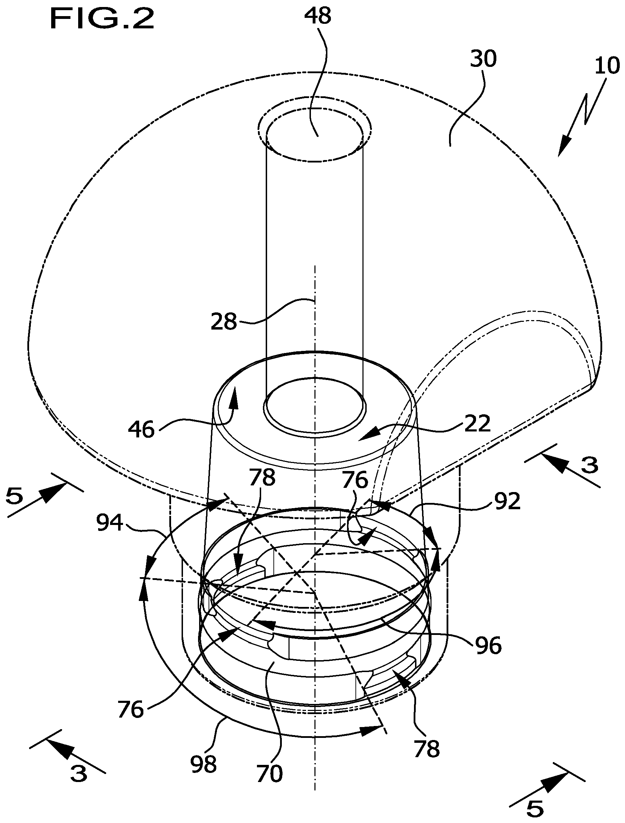

Die ersten und zweiten Rastelemente 76 und 78 sind nicht nur axial zueinander versetzt bezogen auf die Implantatlängsachse 28 angeordnet beziehungsweise ausgebildet, sondern auch in Umfangsrichtung versetzt zueinander, und zwar derart, dass in Umfangsrichtung jedes erste Rastelement 76 zwischen zwei zweiten Rastelementen 78 und jedes zweite Rastelement 78 zwischen zwei ersten Rastelementen 76 angeordnet beziehungsweise ausgebildet ist. Dies ist insbesondere gut in den Figuren 1 und 2 zu erkennen.

Die ersten und zweiten Rastvorsprünge 80 und 82 sind wulstartig von den jeweiligen hohlzylindrischen Abschnitten 66, 68 vorstehend ausgebildet. Die ersten Rastelemente 76 erstrecken sich bezogen auf die Implantatlängsachse 28 jeweils über einen ersten Umfangswinkel 92. Die zweiten Rastelemente 78 erstrecken sich jeweils über einen zweiten Umfangswinkel 94 bezogen auf die Implantatlängsachse 28. Bei dem in den Figuren dargestellten Ausführungsbeispiel ist der erste Umfangswinkel 92 größer als der zweite Umfangswinkel 94.

Der erste Umfangswinkel 92 weist einen Wert in einem Bereich von etwa 30° bis etwa 50° auf. Bei dem in den Figuren dargestellten Ausführungsbeispiel beträgt der Wert des ersten Umfangswinkels 92 etwa 40°.

Der zweite Umfangswinkel 94 weist dagegen einen Wert in einem Bereich von etwa 20° bis etwa 30° auf. Bei dem in den Figuren dargestellten Ausführungsbeispiel beträgt der Wert des zweiten Umfangswinkels 94 etwa 25°.

Die Nut 72 erstreckt sich wie beschrieben zwischen den zwei ersten Rastelementen 76 einerseits und den zweiten Rastelementen 78 andererseits. Die Nut 72 beziehungsweise die Vertiefung 70 bildet einen geschwächten Bereich des zweiten Hülsenabschnitts 58, wodurch eine Elastizität der Hülsenwand 38 in diesem Bereich erhöht wird.

Die ersten Rastelemente 76 erstrecken sich somit gemeinsam nicht über den gesamten Umfang des ersten hohlzylindrischen Abschnitts 66. Dies gilt entsprechend auch für die zweiten Rastelemente 78 bezogen auf den zweiten hohlzylindrischen Abschnitt 68. In Umfangsrichtung werden somit ein erster Bereich 96 zwischen den ersten Rastelementen 76 und ein zweiter Bereich 98 zwischen den zweiten Rastelementen 78 definiert. In den ersten und zweiten Bereichen 96 und 98 sind an der Hülsenwand 38 weder Einschnitte noch Durchbrechungen ausgebildet. Mithin sind also die Bereiche 96 und 98 einschnitt- und durchbrechungsfrei ausgebildet.

Aber nicht nur in den Bereichen 96 und 98 ist die Hülsenwand 38 einschnitt- und durchbrechungsfrei ausgebildet. Vielmehr ist die Hülsenwand 38 vollständig einschnitt- und durchbrechungsfrei ausgebildet. Es sind also beim ersten Probeimplantatteil 10 keine elastischen oder flexiblen Finger ausgebildet, an denen die ersten und zweiten Rastelemente 76 beziehungsweise 78 angeordnet oder ausgebildet sind.

Die ersten und zweiten Rastelemente 76 und 78 sind jeweils in Umfangsrichtung angefast. In axialer Richtung weisen sie einen kantenfreien Verlauf auf. Dieser umfasst jeweils ausgehend von den hohlzylindrischen Wandabschnitten 66 beziehungsweise 68 einen auf die Implantatlängsachse 28 weisenden konkaven, einen sich daran anschließenden konvexen und einen sich daran anschließenden weiteren konkaven Abschnitt auf. Durch diese Ausgestaltung können die Rastelemente 76 und 78 an von der Implantatlängsachse 28 weg weisenden Konusaußenfläche der Verbindungskonen 24, 26 aufgleiten, wenn die Verbindungskonen 24 beziehungsweise 26 in die Konusaufnahme 22 eingeführt werden.

Das erste Probeimplantatteil 10 ist aus einem Kunststoff ausgebildet. Als Kunststoff wird insbesondere Polyphenylensulfon (PPSU) eingesetzt.

Um das erste Probeimplantatteil 10 unter Röntgenkontrolle sichtbar zu machen, enthält der Kunststoff ein Röntgenkontrastmittel. Dabei kann es sich insbesondere um Bariumsulfat handeln.

Bei dem in den Figuren beispielhaft dargestellten Probeimplantatsystem 12 sind die zweiten Probeimplantatteile 14, 16 in Form eines in eine Knochenkavität einsetzbaren Probeschafts 100 beziehungsweise 102 ausgebildet. Alternativ können zweite Probeimplantatteile auch in Form von in den Figuren nicht dargestellten Raspelkörpern ausgebildet sein. Diese können ebenfalls entsprechende Verbindungskonen aufweisen, die analog zu den Verbindungskonen 24, 26 ausgebildet und mit dem ersten Probeimplantatteil 10 wie beschrieben rastend koppelbar sind.

An dieser Stelle sei angemerkt, dass anstelle der in den Figuren beispielhaft dargestellten zweiten Probeimplantatteilen 14 und 16, die lediglich für einen temporären Verbleib im Körper eines Patienten bestimmt sind, auch in Form und Größe identisch ausgebildete Implantatteile, die für einen dauerhaften Verbleib im Körper des Patienten bestimmt sind, im Zusammenwirken mit dem ersten Probeimplantatteil 10 zur Ausbildung erster Probegelenkimplantatteile des modularen Probeimplantatsystems 12 zum Einsatz kommen können. Diese Implantatteile können ebenfalls entsprechende Verbindungskonen aufweisen, die analog zu den Verbindungskonen 24, 26 ausgebildet und mit dem ersten Probeimplantatteil 10 wie beschrieben rastend koppelbar sind. Auf die Darstellung derartiger Implantatteile wurde der Übersichtlichkeit wegen in den Figuren verzichtet, da sie sich wie erläutert in Form und Größe von den zweiten Probeimplantatteilen 14 und 16 nicht unterscheiden.

Das modulare Probeimplantatsystem 12 kann ferner auch ein künstliches Probegelenk 104 beziehungsweise 106 umfassen. Das Probegelenk 104 umfasst dabei den ersten Probegelenkimplantatteil 18 und einen mit diesem gelenkig zusammenwirkenden zweiten Probegelenkimplantatteil 108. Das Probegelenk 106 umfasst den ersten Probegelenkimplantatteil 20 und einen mit diesem gelenkig zusammenwirkenden zweiten Probegelenkimplantatteil 110.

In den Figuren 7 und 9 sind die Probegelenke 104 und 106 beispielhaft als in Form von Probehülftgelenken ausgebildet dargestellt. Die ersten Probegelenkimplantatteile 18, 20 bilden dabei einen Prothesenschaft mit Probegelenkkopf und die zweiten Probegelenkimplantatteile 108, 110 sind in Form einer Probegelenkpfanne 112 beziehungsweise 114 ausgebildet.

Bei dem in den Figuren dargestellten Ausführungsbeispiel des modularen Probeimplantatsystems 12 sind die ersten Probeimplantatteile 10 und die zweiten Probeimplantatteile 14, 16 jeweils einstückig, nämlich monolithisch, ausgebildet.

Die Funktionsweise des modularen Probeimplantatsystems 12, insbesondere die rastende Verbindung zwischen dem ersten Probeimplantatteil 10 den zweiten Probeimplantatteilen 14 beziehungsweise 16 wird nachfolgend kurz erläutert.

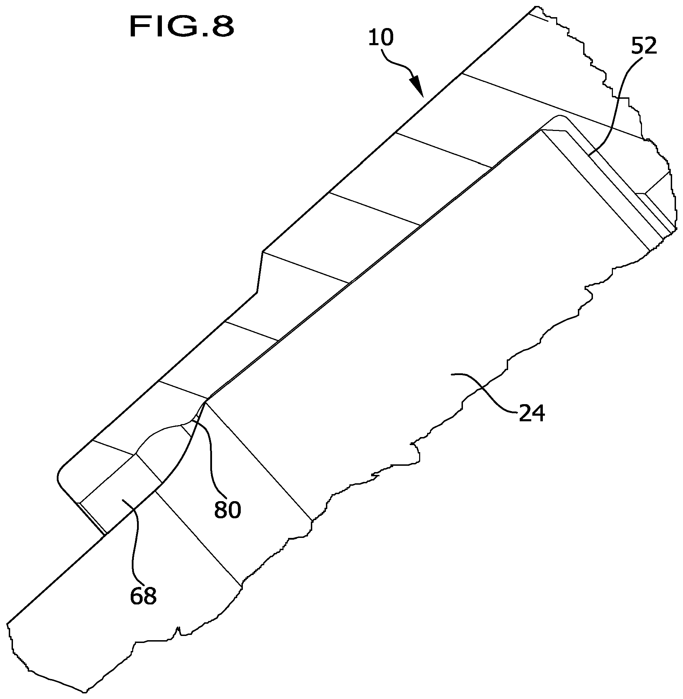

Die Figuren 7 und 8 zeigen das erste Probegelenkimplantatteil 18 beim Koppeln der ersten und zweiten Probeimplantatteile 10 und 14 miteinander. Der Verbindungskonus 24 wird in die Konusaufnahme 22 eingeführt. Er weist eine Länge auf, so dass er mit den ersten Rastelementen 76 zusammenwirken kann. Beim Einführen des Verbindungskonus 24 in die Konusaufnahme 22 verformt sich die Hülsenwand 38 insbesondere im Bereich des zweiten Hülsenabschnitts 58 sowie im Bereich des ersten hohlzylindrischen Abschnitts 66, wenn die ersten Rastelemente 76 außen am Verbindungskonus 24 aufgleiten. Wird das erste Probeimplantatteil 10 weiter auf den Verbindungskonus 24 aufgeschoben, können die ersten Rastelemente 76 eine von der Konusendfläche weg weisende Kante am Verbindungskonus 24 hintergreifen, wodurch die auf das erste Probeimplantatteil 10 ausgeübte Vorschubkraft zu einer ruckartigen Beschleunigung desselben führt, so dass der Konusboden 46 gegen die Konusendfläche 52 ungebremst anschlagen und so ein Klick- oder Schnappgeräusch verursachen kann.

Wird das erste Probeimplantatteil 10 mit dem Verbindungskonus 26 des zweiten Probeimplantatteils 16 in Eingriff gebracht, so gleiten die ersten Rastelemente 76 außen am Verbindungskonus 26 auf. Sie führen dabei zu einer Verformung der Hülsenwand 38 im Bereich des zweiten Hülsenabschnitts 58 sowie des ersten hohlzylindrischen Abschnitts 66. Sobald die zweiten Rastelemente 78 den längeren Verbindungskonus 26 hintergreifen können, führt dies wiederum aufgrund der einwirkenden Vorschubkraft auf das erste Probeimplantatteil 10 zu einer beschleunigten Bewegung desselben in Richtung auf den Verbindungskonus 26 hin, so dass der Konusboden 46 auch gegen die Konusendfläche 54 anschlagen und ein entsprechendes Klick- oder Anschlaggeräusch hervorrufen kann.

Die Vertiefung 70, die die Hülsenwand 38 etwas schwächt und damit elastischer macht, verbessert eine elastische Verformung und damit auch ein Zurückverschwenken der zweiten Rastelemente 78 beim Hintergreifen des Verbindungskonus 26 in ihre unverformte Ausgangsstellung. So ist - trotz der außen am Verbindungskonus 26 entlanggleitenden ersten Rastelemente 76 - eine hinreichende Beschleunigung des ersten Probeimplantatteils 10 zum Hervorrufen eines Klickgeräuschs möglich.

Das beschriebene modulare Probeimplantatsystem 12, insbesondere das erste Probeimplantatteil 10, ermöglicht einem Operateur eine einfache Handhabung desselben und insbesondere ein sicheres Verbinden der ersten und zweiten Probeimplantatteile 10 beziehungsweise 14, 16 oder alternativ des ersten Probeimplantatteils 10 mit einem Implantatteil miteinander, denn wie erläutert erhält der Operateur eine akustische Rückmeldung sowohl beim Koppeln des ersten Probeimplantatteils 10 mit dem kürzeren Verbindungskonus 24 als auch mit dem längeren Verbindungskonus 26.

### Bezugszeichenlisten

- 10: erstes Probeimplantatteil
- 12: modulares Probeimplantatsystem
- 14: zweites Probeimplantatteil
- 16: zweites Probeimplantatteil
- 18: erstes Probegelenkimplantatteil
- 20: erstes Probegelenkimplantatteil
- 22: Konusaufnahme
- 24: Verbindungskonus
- 26: Verbindungskonus
- 28: Implantatlängsachse
- 30: Probegelenkkopf
- 32: Kugeloberfläche
- 34: Unterseite
- 36: Aufnahmehülse
- 38: Hülsenwand
- 40: Einführöffnung
- 42: Einführöffnungsrand
- 44: Randebene
- 46: Konusboden
- 48: Durchbrechung
- 50: Anschlagfläche
- 52: Konusendfläche
- 54: Konusendfläche
- 56: erster Hülsenabschnitt
- 58: zweiter Hülsenabschnitt
- 60: erste Wanddicke
- 62: zweite Wanddicke
- 64: Innenwandfläche
- 66: erster hohlzylindrischer Abschnitt
- 68: zweiter hohlzylindrischer Abschnitt
- 70: Vertiefung
- 72: Nut
- 74: Nutebene
- 76: erstes Rastelement
- 78: zweites Rastelement
- 80: erster Rastvorsprung
- 82: zweiter Rastvorsprung
- 84: erste Rastelementebene
- 86: zweite Rastelementebene
- 88: erster Abstand
- 90: zweiter Abstand
- 92: erster Umfangswinkel
- 94: zweiter Umfangswinkel
- 96: erster Bereich
- 98: zweiter Bereich
- 100: Probeschaft
- 102: Probeschaft
- 104: Probegelenk
- 106: Probegelenk
- 108: zweites Probegelenkimplantatteil
- 110: zweites Probegelenkimplantatteil
- 112: Probegelenkpfanne
- 114: Probegelenkpfanne

## Patentansprüche

1. Modulares Probeimplantatsystem (12) umfassend mindestens ein erstes Probeimplantatteil (10), welches ausgebildet ist zum lösbaren Verbinden mit mindestens einem zweiten Probeimplantatteil (14, 16) und/oder einem Implantatteil zur Ausbildung mindestens eines ersten Probegelenkimplantatteils (18, 20), wobei das mindestens eine erste Probeimplantatteil (10) eine Konusaufnahme (22) zum Aufnehmen eines Verbindungskonus (24, 26) des mindestens einen zweiten Probeimplantatteils (14, 16) oder des mindestens einen Implantatteils umfasst, wobei die Konusaufnahme (22) eine Implantatlängsachse (28) definiert, wobei das mindestens eine erste Probeimplantatteil (10) mindestens zwei einander zugeordnete erste Rastelemente (76) und mindestens zwei einander zugeordnete zweite Rastelemente (78) umfasst, wobei die mindestens zwei ersten Rastelemente (76) einerseits und die mindestens zwei zweiten Rastelemente (78) andererseits in der Konusaufnahme (22) bezogen auf die Implantatlängsachse (28) axial versetzt angeordnet oder ausgebildet sind zum rastenden Hintergreifen unterschiedlich langer Verbindungskonen (24, 26) zweiter Probeimplantatteile (14, 16) oder Implantatteile, wobei das mindestens eine erste Probeimplantatteil (10) eine die Konusaufnahme (22) definierende Aufnahmehülse (36) umfasst, wobei die Aufnahmehülse (36) eine die Implantatlängsachse (28) umgebende und die Konusaufnahme (22) begrenzende Hülsenwand (38) umfasst, **dadurch gekennzeichnet,**
**dass** die Hülsenwand (38) mindestens in einem ersten Bereich (96) zwischen den mindestens zwei ersten Rastelementen (78) und mindestens in einem zweiten Bereich (98) zwischen den mindestens zwei zweiten Rastelementen (78) einschnitt- und/oder durchbrechungsfrei ausgebildet ist.

2. Modulares Probeimplantatsystem nach Anspruch 1, **dadurch gekennzeichnet, dass** die Hülsenwand (38) vollständig einschnitt- und/oder durchbrechungsfrei ausgebildet ist.

3. Modulares Probeimplantatsystem nach Anspruch 1 oder 2, **dadurch gekennzeichnet, dass** die Konusaufnahme (22) eine Einführöffnung (40) zum Einführen des Verbindungskonus (24, 26) umfasst und dass die Einführöffnung (40) von einem kreisringförmigen, unterbrechungsfreien Einführöffnungsrand (42) begrenzt ist,
wobei insbesondere der Einführöffnungsrand (42) eine Randebene (44) definiert und dass sich die Randebene (44) quer, insbesondere senkrecht, zur Implantatlängsachse (28) erstreckt.

4. Modulares Probeimplantatsystem nach einem der voranstehenden Ansprüche, **dadurch gekennzeichnet, dass** sich die Konusaufnahme (22) in Richtung auf einen Konusboden (46) hin konisch verjüngt und dass sich der Konusboden (46) quer, insbesondere senkrecht, zur Implantatlängsachse (28) erstreckt,
wobei insbesondere
a) am mindestens einen ersten Probeimplantatteil (10) eine Durchbrechung (48) ausgebildet ist und dass die Durchbrechung (48) den Konusboden (46) durchsetzt,
wobei insbesondere die Durchbrechung (48) koaxial zur Implantatlängsachse (28) angeordnet oder ausgebildet ist,
und/oder
b) der Konusboden (46) eine in Richtung auf die Einführöffnung (40) hin weisende Anschlagfläche (50) für eine Konusendfläche (52, 54) des Verbindungskonus (24, 26) bildet, wobei die Anschlagfläche (50) insbesondere ringförmig ausgebildet ist,
und/oder
c) ein erster Abstand (88) der mindestes zwei ersten Rastelemente (76) vom Konusboden (46) kleiner ist als ein zweiter Abstand (90) der mindestens zwei zweiten Rastelemente (78) vom Konusboden (46).

5. Modulares Probeimplantatsystem nach einem der voranstehenden Ansprüche, **dadurch gekennzeichnet, dass**
a) sich die mindestens zwei ersten Rastelemente (76) bezogen auf die Implantatlängsachse (28) in Umfangsrichtung erstrecken
und/oder
b) die mindestens zwei ersten Rastelemente (76) gleichmäßig über einen Umfang der Hülsenwand (38) verteilt angeordnet oder ausgebildet sind
und/oder
c) sich die mindestens zwei zweiten Rastelemente (78) bezogen auf die Implantatlängsachse (28) in Umfangsrichtung erstrecken und/oder
d) die mindestens zwei zweiten Rastelemente (78) gleichmäßig über einen Umfang der Hülsenwand (38) verteilt angeordnet oder ausgebildet sind.

6. Modulares Probeimplantatsystem nach einem der voranstehenden Ansprüche, **dadurch gekennzeichnet, dass**
a) die mindestens zwei ersten Rastelemente (76) eine erste Rastelementebene (84) definieren und dass sich die erste Rastelementebene (84) quer, insbesondere senkrecht, zur Implantatlängsachse (28) erstreckt
und/oder
b) die mindestens zwei zweiten Rastelemente (78) eine zweite Rastelementebene (86) definieren und dass sich die zweite Rastelementebene (86) quer, insbesondere senkrecht, zur Implantatlängsachse (28) erstreckt
und/oder
c) die mindestens zwei ersten Rastelemente (76) in Form von in Richtung auf die Implantatlängsachse (28) hin weisenden ersten Rastvorsprüngen (80) ausgebildet sind
und/oder
d) die mindestens zwei zweiten Rastelemente (78) in Form von in Richtung auf die Implantatlängsachse (28) hin weisenden zweiten Rastvorsprüngen (82) ausgebildet sind.

7. Modulares Probeimplantatsystem nach einem der voranstehenden Ansprüche, **dadurch gekennzeichnet, dass** die mindestens zwei ersten Rastelemente (76) und die mindestens zwei zweiten Rastelemente (78) in Umfangsrichtung versetzt zueinander angeordnet oder ausgebildet sind derart, dass in Umfangsrichtung jedes erste Rastelement (76) zwischen zwei zweiten Rastelementen (78) und jedes zweite Rastelement (78) zwischen zwei ersten Rastelementen (76) angeordnet oder ausgebildet ist.

8. Modulares Probeimplantatsystem nach einem der voranstehenden Ansprüche, **dadurch gekennzeichnet, dass**
a) zwei erste Rastelemente (76) vorgesehen sind und dass die zwei ersten Rastelemente (76) bezogen auf die Implantatlängsachse (28) einander diametral gegenüberliegend angeordnet oder ausgebildet sind
und/oder
b) zwei zweite Rastelemente (78) vorgesehen sind und dass die zwei zweiten Rastelemente (78) bezogen auf die Implantatlängsachse (28) einander diametral gegenüberliegend angeordnet oder ausgebildet sind.

9. Modulares Probeimplantatsystem nach einem der voranstehenden Ansprüche, **dadurch gekennzeichnet, dass** sich die mindestens zwei ersten Rastelemente (76) über einen ersten Umfangswinkel (92) bezogen auf die Implantatlängsachse (28) erstrecken, dass sich die mindestens zwei zweiten Rastelemente (78) über einen zweiten Umfangswinkel (94) bezogen auf die Implantatlängsachse (28) erstrecken und dass der erste Umfangswinkel (92) größer ist als der zweite Umfangswinkel (94), wobei insbesondere der erste Umfangswinkel (92) einen Wert in einem Bereich von etwa 30° bis etwa 50° aufweist, insbesondere in einem Bereich von etwa 35° bis etwa 45°, und dass der zweite Umfangswinkel (94) einen Wert in einem Bereich von etwa 20° bis etwa 30° aufweist, insbesondere in einem Bereich von etwa 22° bis etwa 28°.

10. Modulares Probeimplantatsystem nach einem der voranstehenden Ansprüche, **dadurch gekennzeichnet, dass** die Konusaufnahme (22) eine Innenwandfläche (64) definiert, dass an der Innenwandfläche (64) ein erster, bezogen auf die Implantatlängsachse (28) hohlzylindrischer Abschnitt (66) ausgebildet ist und dass die mindestens zwei ersten Rastelemente (76) am ersten hohlzylindrischen Abschnitt (66) ausgebildet sind, wobei insbesondere an der Innenwandfläche (64) ein zweiter, bezogen auf die Implantatlängsachse (28) hohlzylindrischer Abschnitt (68) ausgebildet ist und dass die mindestens zwei zweiten Rastelemente (78) am zweiten hohlzylindrischen Abschnitt (68) ausgebildet sind.

11. Modulares Probeimplantatsystem nach einem der voranstehenden Ansprüche, **dadurch gekennzeichnet, dass** an der Konusaufnahme (22) eine bezogen auf die Implantatlängsachse (28) umlaufende Nut (72) angeordnet oder ausgebildet ist und dass die Nut (72) zwischen den mindestens zwei ersten Rastelementen (76) einerseits und den mindestens zwei zweiten Rastelementen (78) andererseits angeordnet oder ausgebildet ist,
wobei insbesondere
a) die Nut (72) in Richtung auf die Implantatlängsachse (28) hin weisend konkav gekrümmt ist
und/oder
b) die Nut (72) eine Nutebene (74) definiert und dass die Nutebene (74) quer, insbesondere senkrecht, zur Implantatlängsachse (28) verläuft
und/oder
c) sich die Nut (72) zwischen dem ersten hohlzylindrischen Abschnitt (66) und dem zweiten hohlzylindrischen Abschnitt (68) erstreckt und/oder
d) die Nut (72) direkt an den ersten hohlzylindrischen Abschnitt (66) und/oder direkt an den zweiten hohlzylindrischen Abschnitt (68) angrenzt.

12. Modulares Probeimplantatsystem nach einem der voranstehenden Ansprüche, **dadurch gekennzeichnet, dass** die Hülsenwand (38) einen ersten Hülsenabschnitt (56) und einen sich daran anschließenden zweiten Hülsenabschnitt (58) umfasst, dass sich die Konusaufnahme (22) vom zweiten Hülsenabschnitt (58) in Richtung auf den ersten Hülsenabschnitt (56) verjüngt, dass die mindestens zwei ersten und zweiten Rastelemente (76, 78) ausschließlich am zweiten Hülsenabschnitt (58) angeordnet oder ausgebildet sind und dass eine erste Wanddicke (60) des ersten Hülsenabschnitts (56) größer ist als eine zweite Wanddicke (62) des zweiten Hülsenabschnitts (58), insbesondere mindestens etwa 50% größer.

13. Modulares Probeimplantatsystem nach einem der voranstehenden Ansprüche, **dadurch gekennzeichnet, dass** das mindestens eine erste Probeimplantatteil (10) aus einem Kunststoff ausgebildet ist,
wobei insbesondere der Kunststoff Polyphenylensulfon (PPSU) ist oder enthält.

14. Modulares Probeimplantatsystem nach einem der voranstehenden Ansprüche, **dadurch gekennzeichnet, dass** das modulare Probeimplantatsystem (12) mindestens ein zweites Probeimplantatteil (14, 16) oder mindestens ein Implantatteil umfasst und dass das mindestens eine zweite Probeimplantatteil (14, 16) oder das mindestens eine Implantatteil einen in die Konusaufnahme (22) einführbaren Verbindungskonus (24, 26) umfasst,
wobei insbesondere
a) das modulare Probeimplantatsystem (12) mindestens zwei zweite Probeimplantatteile (14, 16) oder mindestens zwei Implantatteile mit unterschiedlich langen Verbindungskonen (24, 26) umfasst und/oder
b) das mindestens eine zweite Probeimplantatteil (14, 16) in Form eines in eine Knochenkavität einsetzbaren Probeschafts (100, 102) oder in Form eines Raspelkörpers ausgebildet ist.

15. Modulares Probeimplantatsystem nach einem der voranstehenden Ansprüche, **dadurch gekennzeichnet, dass**
a) das mindestens eine erste Probeimplantatteil (10) einen kugeligen oder kugelförmigen Probegelenkkopf (30) aufweist,
wobei insbesondere der Probegelenkkopf (30) einen bezogen auf die Implantatlängsachse (28) rotationssymmetrischen Ausschnitt einer Kugeloberfläche (32) aufweist,
wobei weiter insbesondere die Durchbrechung (48) die Konusaufnahme (22) und die Kugeloberfläche (32) fluidwirksam verbindet,
und/oder
b) das modulare Probeimplantatsystem (12) mindestens ein künstliches Probegelenk (104, 106) mit einem ersten Probegelenkimplantatteil (18, 20) und einem mit diesem gelenkig zusammenwirkenden zweiten Probegelenkimplantatteil (108, 110) umfasst, wobei insbesondere das künstliche Probegelenk (104, 106) in Form eines Probehüftgelenks ausgebildet ist, wobei das erste Probegelenkimplantatteil (18, 20) einen Prothesenschaft mit Probegelenckopf bildet und wobei das zweite Probegelenkimplantatteil (108, 110) in Form einer Probegelenkpfanne (112, 114) ausgebildet ist, und/oder
c) das mindestens eine erste Probeimplantatteil (10) und/oder das mindestens eine zweite Probeimplantatteil (14, 16) oder das Implantatteil einstückig, insbesondere monolithisch, ausgebildet sind.

## Claims

1. Modular trial implant system (12) comprising at least one first trial implant part (10), which is configured to releasably connect to at least one second trial implant part (14, 16) and/or an implant part to form at least one first trial joint implant part (18, 20), wherein the at least one first trial implant part (10) comprises a cone receptacle (22) for accommodating a connecting cone (24, 26) of the at least one second trial implant part (14, 16) or the at least one implant part, wherein the cone receptacle (22) defines an implant longitudinal axis (28), wherein the at least one first trial implant part (10) comprises at least two first latching elements (76) associated with one another and at least two second latching elements (78) associated with one another, wherein the at least two first latching elements (76) on the one hand and the at least two second latching elements (78) on the other hand are arranged or formed axially offset in the cone receptacle (22) relative to the implant longitudinal axis (28) for latchingly engaging behind differently long connecting cones (24, 26) of second trial implant parts (14, 16) or implant parts, wherein the at least one first trial implant part (10) comprises a receiving sleeve (36) defining the cone receptacle (22), wherein the receiving sleeve (36) comprises a sleeve wall (38) that surrounds the implant longitudinal axis (28) and delimits the cone receptacle (22), **characterized in that** the sleeve wall (38) is of indentation-free and/or perforation-free configuration at least in a first region (96) between the at least two first latching elements (78) and at least in a second region (98) between the at least two second latching elements (78).

2. Modular trial implant system in accordance with claim 1, **characterized in that** the sleeve wall (38) is completely of indentation-free and/or perforation-free configuration.

3. Modular trial implant system in accordance with claim 1 or 2,
**characterized in that** the cone receptacle (22) comprises an insertion opening (40) for inserting the connecting cone (24, 26) and **in that** the insertion opening (40) is delimited by a circular ring-shaped, uninterrupted insertion opening rim (42),
wherein, in particular, the insertion opening rim (42) defines a rim plane (44) and **in that** the rim plane (44) extends transversely, in particular perpendicularly, to the implant longitudinal axis (28).

4. Modular trial implant system in accordance with any one of the preceding claims, **characterized in that** the cone receptacle (22) conically tapers in the direction toward a cone base (46) and **in that** the cone base (46) extends transversely, in particular perpendicularly, to the implant longitudinal axis (28),
wherein, in particular,
a) a perforation (48) is formed on the at least one first trial implant part (10) and **in that** the perforation (48) passes through the cone base (46),
wherein, in particular, the perforation (48) is arranged or formed coaxially to the implant longitudinal axis (28),
and/or
b) the cone base (46) forms a stop face (50) for a cone end face (52, 54) of the connecting cone (24, 26), said stop face pointing in the direction toward the insertion opening (40), wherein the stop face (50) is, in particular, of annular configuration,
and/or
c) a first distance (88) of the at least two first latching elements (76) from the cone base (46) is smaller than a second distance (90) of the at least two second latching elements (78) from the cone base (46).

5. Modular trial implant system in accordance with any one of the preceding claims, **characterized in that**
a) the at least two first latching elements (76) extend in the circumferential direction relative to the implant longitudinal axis (28)
and/or
b) the at least two first latching elements (76) are arranged or formed evenly distributed over a circumference of the sleeve wall (38) and/or
c) the at least two second latching elements (78) extend in the circumferential direction relative to the implant longitudinal axis (28)
and/or
d) the at least two second latching elements (78) are arranged or formed evenly distributed over a circumference of the sleeve wall (38).

6. Modular trial implant system in accordance with any one of the preceding claims, **characterized in that**
a) the at least two first latching elements (76) define a first latching element plane (84) and **in that** the first latching element plane (84) extends transversely, in particular perpendicularly, to the implant longitudinal axis (28)
and/or
b) the at least two second latching elements (78) define a second latching element plane (86) and **in that** the second latching element plane (86) extends transversely, in particular perpendicularly, to the implant longitudinal axis (28)
and/or
c) the at least two first latching elements (76) are configured in the form of first latching projections (80) pointing in the direction toward the implant longitudinal axis (28)
and/or
d) the at least two second latching elements (78) are configured in the form of second latching projections (82) pointing in the direction toward the implant longitudinal axis (28).

7. Modular trial implant system in accordance with any one of the preceding claims, **characterized in that** the at least two first latching elements (76) and the at least two second latching elements (78) are arranged or formed offset from one another in the circumferential direction in such a way that, in the circumferential direction, each first latching element (76) is arranged or formed between two second latching elements (78) and each second latching element (78) is arranged of formed between two first latching elements (76).

8. Modular trial implant system in accordance with any one of the preceding claims, **characterized in that**
a) two first latching elements (76) are provided and **in that** the two first latching elements (76) are arranged or formed diametrically opposed to one another relative to the implant longitudinal axis (28)
and/or
b) two second latching elements (78) are provided and **in that** the two second latching elements (78) are arranged or formed diametrically opposed to one another relative to the implant longitudinal axis (28).

9. Modular trial implant system in accordance with any one of the preceding claims, **characterized in that** the at least two first latching elements (76) extend over a first circumferential angle (92) relative to the implant longitudinal axis (28), **in that** the at least two second latching elements (78) extend over a second circumferential angle (94) relative to the implant longitudinal axis (28), and **in that** the first circumferential angle (92) is greater than the second circumferential angle (94),
wherein, in particular, the first circumferential angle (92) has a value in a range of about 30° to about 50°, in particular in a range of about 35° to about 45°, and **in that** the second circumferential angle (94) has a value in a range of about 20° to about 30°, in particular in a range of about 22° to about 28°.

10. Modular trial implant system in accordance with any one of the preceding claims, **characterized in that** the cone receptacle (22) defines an inner wall surface (64), **in that** formed on the inner wall surface (64) is a first portion (66) that is hollow-cylindrical relative to the implant longitudinal axis (28), and **in that** the at least two first latching elements (76) are formed on the first hollow-cylindrical portion (66),
wherein, in particular, formed on the inner wall surface (64) is a second portion (68) that is hollow-cylindrical relative to the implant longitudinal axis (28) and **in that** the at least two second latching elements (78) are formed on the second hollow-cylindrical portion (68).

11. Modular trial implant system in accordance with any one of the preceding claims, **characterized in that** a circumferential groove (72) relative to the implant longitudinal axis (28) is arranged or formed on the cone receptacle (22) and **in that** the groove (72) is arranged or formed between the at least two first latching elements (76) on the one hand and the at least two second latching elements (78) on the other hand, wherein, in particular,
a) the groove (72) is concavely curved pointing in the direction toward the implant longitudinal axis (28)
and/or
b) the groove (72) defines a groove plane (74) and **in that** the groove plane (74) extends transversely, in particular perpendicularly, to the implant longitudinal axis (28)
and/or
c) the groove (72) extends between the first hollow-cylindrical portion (66) and the second hollow-cylindrical portion (68)
and/or
d) the groove (72) directly adjoins the first hollow-cylindrical portion (66) and/or directly adjoins the second hollow-cylindrical portion (68).

12. Modular trial implant system in accordance with any one of the preceding claims, **characterized in that** the sleeve wall (38) comprises a first sleeve portion (56) and an adjoining second sleeve portion (58), **in that** the cone receptacle (22) tapers from the second sleeve portion (58) in the direction toward the first sleeve portion (56), **in that** the at least two first and second latching elements (76, 78) are arranged or formed exclusively on the second sleeve portion (58), and **in that** a first wall thickness (60) of the first sleeve portion (56) is greater than a second wall thickness (62) of the second sleeve portion (58), in particular at least about 50% greater.

13. Modular trial implant system in accordance with any one of the preceding claims, **characterized in that** the at least one first trial implant part (10) is made of a plastic,
wherein, in particular, the plastic is or contains polyphenylene sulfone (PPSU).

14. Modular trial implant system in accordance with any one of the preceding claims, **characterized in that** the modular trial implant system (12) comprises at least one second trial implant part (14, 16) or at least one implant part and **in that** the at least one second trial implant part (14, 16) or the at least one implant part comprises a connecting cone (24, 26) that is insertable into the cone receptacle (22),
wherein, in particular,
a) the modular trial implant system (12) comprises at least two second trial implant parts (14, 16) or at least two implant parts with connecting cones (24, 26) of different lengths
and/or
b) the at least one second trial implant part (14, 16) is configured in the form of a trial stem (100, 102) that is insertable into a bone cavity or in the form of a rasp body.

15. Modular trial implant system in accordance with any one of the preceding claims, **characterized in that**
a) the at least one first trial implant part (10) has a spherical or ball-shaped trial joint head (30),
wherein, in particular, the trial joint head (30) has a rotationally symmetrical portion of a spherical surface (32) relative to the implant longitudinal axis (28),
wherein, further in particular, the perforation (48) fluidically connects the cone receptacle (22) and the spherical surface (32),
and/or
b) the modular trial implant system (12) comprises at least one artificial trial joint (104, 106) with a first trial joint implant part (18, 20) and a second trial joint implant part (108, 110) that cooperates with said first trial joint implant part in a jointed manner,
wherein, in particular, the artificial trial joint (104, 106) is configured in the form of a trial hip joint, wherein the first trial joint implant part (18, 20) forms a prosthesis shaft with a trial joint head, and wherein the second trial joint implant part (108, 110) is configured in the form of a trial joint socket (112, 114),
and/or
c) the at least one first trial implant part (10) and/or the at least one second trial implant part (14, 16) or the implant part are of one-piece, in particular monolithic, configuration.

## Revendications

1. Système modulaire d'implant d'essai (12) comprenant au moins une première partie d'implant d'essai (10) qui est conçue pour être reliée de manière amovible à au moins une deuxième partie d'implant d'essai (14, 16) et/ou à une partie d'implant pour former au moins une première partie articulaire d'implant d'essai (18, 20), dans lequel l'au moins une première partie d'implant d'essai (10) comprend un logement conique (22) pour recevoir un cône de liaison (24, 26) de l'au moins une deuxième partie d'implant d'essai (14, 16) ou de l'au moins une partie d'implant, le logement conique (22) définissant un axe longitudinal d'implant (28), l'au moins une première partie d'implant d'essai (10) comprenant au moins deux premiers éléments d'encliquetage (76) associés l'un à l'autre et au moins deux deuxièmes éléments d'encliquetage (78) associés l'un à l'autre, les au moins deux premiers éléments d'encliquetage (76) d'une part et les au moins deux deuxièmes éléments d'encliquetage (78) d'autre part étant agencés ou réalisés dans le logement conique (22) de manière décalée axialement par rapport à l'axe longitudinal d'implant (28) pour l'engagement par l'arrière par encliquetage de cônes de liaison (24, 26) de longueurs différentes de deuxièmes parties d'implant d'essai (14, 16) ou de parties d'implant, dans lequel l'au moins une première partie d'implant d'essai (10) comprend une douille de réception (36) définissant le logement conique (22), la douille de réception (36) comprenant une paroi de douille (38) entourant l'axe longitudinal d'implant (28) et délimitant le logement conique (22), **caractérisée en ce que** la paroi de douille (38) est formée sans incision et/ou perforation dans une première zone (96) entre les au moins deux premiers éléments d'encliquetage (78) et au moins dans une deuxième zone (98) entre les au moins deux deuxièmes éléments d'encliquetage (78).

2. Système modulaire d'implant d'essai selon la revendication 1, **caractérisé en ce que** la paroi de douille (38) est entièrement exempte d'incisions et/ou de perforations.

3. Système modulaire d'implant d'essai selon la revendication 1 ou 2, **caractérisé en ce que** le logement conique (22) comprend une ouverture d'insertion (40) pour l'insertion du cône de liaison (24, 26) et **en ce que** l'ouverture d'insertion (40) est délimitée par un bord d'ouverture d'insertion (42) en forme d'anneau circulaire, sans interruption,
le bord d'ouverture d'insertion (42) définissant en particulier un plan de bord (44) et **en ce que** le plan de bord (44) s'étend transversalement, en particulier perpendiculairement, à l'axe longitudinal d'implant (28).

4. Système modulaire d'implant d'essai selon l'une des revendications précédentes, **caractérisé en ce que** le logement conique (22) se rétrécit en cône en direction d'un fond du cône (46) et **en ce que** le fond du cône (46) s'étend transversalement, en particulier perpendiculairement, à l'axe longitudinal d'implant (28),
dans lequel, en particulier,
a) une incision (48) est formée sur au moins une première partie d'implant d'essai (10) et **en ce que** l'incision (48) traverse le fond du cône (46),
l'incision (48) étant en particulier agencée ou réalisée coaxialement à l'axe longitudinal d'implant (28),
et/ou
b) le fond du cône (46) forme une surface de butée (50) orientée vers l'ouverture d'insertion (40) pour une surface d'extrémité de cône (52, 54) du cône de liaison (24, 26), la surface de butée (50) étant en particulier de forme annulaire,
et/ou
c) une première distance (88) des au moins deux premiers éléments d'encliquetage (76) par rapport au fond du cône (46) est inférieure à une deuxième distance (90) des au moins deux deuxièmes éléments d'encliquetage (78) par rapport au fond du cône (46).

5. Système modulaire d'implant d'essai selon l'une quelconque des revendications précédentes, **caractérisé en ce que**
a) les au moins deux premiers éléments d'encliquetage (76) s'étendent dans la direction circonférentielle par rapport à l'axe longitudinal d'implant (28)
et/ou
b) les au moins deux premiers éléments d'encliquetage (76) sont agencés ou formés de manière uniformément répartie sur une périphérie de la paroi de douille (38)
and/or
c) les au moins deux deuxièmes éléments d'encliquetage (78) s'étendent dans la direction circonférentielle par rapport à l'axe longitudinal d'implant (28)
et/ou
d) les au moins deux deuxièmes éléments d'encliquetage (78) sont agencés ou formés de manière uniformément répartie sur une périphérie de la paroi de douille (38).

6. Système modulaire d'implant d'essai selon l'une quelconque des revendications précédentes, **caractérisé en ce que**
a) les au moins deux premiers éléments d'encliquetage (76) définissent un premier plan d'éléments d'encliquetage (84) et **en ce que** le premier plan d'éléments d'encliquetage (84) s'étend transversalement, en particulier perpendiculairement, à l'axe longitudinal de l'implant (28)
et/ou
b) les au moins deux deuxièmes éléments d'encliquetage (78) définissent un deuxième plan d'éléments d'encliquetage (86) et **en ce que** le deuxième plan d'éléments d'encliquetage (86) s'étend transversalement, en particulier perpendiculairement, à l'axe longitudinal de l'implant (28)
et/ou
c) les au moins deux premiers éléments d'encliquetage (76) sont réalisés sous la forme de premières saillies d'encliquetage (80) orientées en direction de l'axe longitudinal de l'implant (28)
et/ou
d) les au moins deux deuxièmes éléments d'encliquetage (78) sont réalisés sous la forme de deuxièmes saillies d'encliquetage (82) orientées en direction de l'axe longitudinal de l'implant (28).

7. Système modulaire d'implant d'essai selon l'une des revendications précédentes, **caractérisé en ce que** les au moins deux premiers éléments d'encliquetage (76) et les au moins deux deuxièmes éléments d'encliquetage (78) sont agencés ou formés de manière décalée l'un par rapport à l'autre dans la direction circonférentielle, de telle sorte que, dans la direction circonférentielle, chaque premier élément d'encliquetage (76) est agencé ou formé entre deux deuxièmes éléments d'encliquetage (78) et chaque deuxième élément d'encliquetage (78) est agencé ou formé entre deux premiers éléments d'encliquetage (76).

8. Système modulaire d'implant d'essai selon l'une quelconque des revendications précédentes, **caractérisé en ce que**
a) deux premiers éléments d'encliquetage (76) sont prévus et **en ce que** les deux premiers éléments d'encliquetage (76) sont agencés ou réalisés de manière diamétralement opposée l'un à l'autre par rapport à l'axe longitudinal de l'implant (28)
et/ou
b) deux deuxièmes éléments d'encliquetage (78) sont prévus et **en ce que** les deux deuxièmes éléments d'encliquetage (78) sont agencés ou formés de manière diamétralement opposée l'un à l'autre par rapport à l'axe longitudinal de l'implant (28).

9. Système modulaire d'implant d'essai selon l'une des revendications précédentes, **caractérisé en ce que** les au moins deux premiers éléments d'encliquetage (76) s'étendent sur un premier angle circonférentiel (92) par rapport à l'axe longitudinal de l'implant (28), **en ce que** les au moins deux deuxièmes éléments d'encliquetage (78) s'étendent sur un deuxième angle circonférentiel (94) par rapport à l'axe longitudinal de l'implant (28) et **en ce que** le premier angle circonférentiel (92) est supérieur au deuxième angle circonférentiel (94),
dans lequel, en particulier, le premier angle circonférentiel (92) présente une valeur située dans une plage d'environ 30° à environ 50°, en particulier dans une plage d'environ 35° à environ 45°, et **en ce que** le deuxième angle circonférentiel (94) présente une valeur située dans une plage d'environ 20° à environ 30°, en particulier dans une plage d'environ 22° à environ 28°.

10. Système modulaire d'implant d'essai selon l'une des revendications précédentes, **caractérisé en ce que** le logement conique (22) définit une surface de paroi intérieure (64), **en ce qu'**une première partie cylindrique creuse (66) par rapport à l'axe longitudinal de l'implant (28) est formée sur la surface de paroi intérieure (64) et **en ce que** les au moins deux premiers éléments d'encliquetage (76) sont formés sur la première partie cylindrique creuse (66),
une deuxième partie cylindrique creuse (68) par rapport à l'axe longitudinal de l'implant (28) étant formée en particulier sur la surface de paroi intérieure (64), et **en ce que** les au moins deux deuxièmes éléments d'encliquetage (78) sont formés sur la deuxième partie cylindrique creuse (68).

11. Système modulaire d'implant d'essai selon l'une des revendications précédentes, **caractérisé en ce qu'**une rainure (72) périphérique par rapport à l'axe longitudinal de l'implant (28) est agencée ou formée sur le logement conique (22) et **en ce que** la rainure (72) est agencée ou formée entre les au moins deux premiers éléments d'encliquetage (76) d'une part et les au moins deux deuxièmes éléments d'encliquetage (78) d'autre part,
dans lequel, en particulier,
a) la rainure (72) est incurvée de manière concave en direction de l'axe longitudinal de l'implant (28)
et/ou
b) la rainure (72) définit un plan de rainure (74) et le plan de rainure (74) s'étend transversalement, en particulier perpendiculairement, à l'axe longitudinal de l'implant (28)
et/ou
c) la rainure (72) s'étend entre la première partie cylindrique creuse (66) et la deuxième partie cylindrique creuse (68)
et/ou
d) la rainure (72) est directement adjacente à la première partie cylindrique creuse (66) et/ou directement adjacente à la deuxième partie cylindrique creuse (68).

12. Système modulaire d'implant d'essai selon l'une des revendications précédentes, **caractérisé en ce que** la paroi de douille (38) comprend une première partie de douille (56) et une deuxième partie de douille (58) qui s'y raccorde, **en ce que** le logement conique (22) se rétrécit à partir de la deuxième partie de douille (58) en direction de la première partie de douille (56), **en ce que** les au moins deux premiers et deuxièmes éléments d'encliquetage (76, 78) sont agencés ou formés exclusivement sur la deuxième partie de douille (58) et **en ce qu'**une première épaisseur de paroi (60) de la première partie de douille (56) est supérieure à une deuxième épaisseur de paroi (62) de la deuxième partie de douille (58), en particulier au moins environ 50 % supérieure.

13. Système modulaire d'implant d'essai selon l'une des revendications précédentes, **caractérisé en ce que** l'au moins une première partie d'implant d'essai (10) est réalisée en matière plastique,
dans lequel, en particulier, la matière plastique est ou contient de la polyphénylène sulfone (PPSU).

14. Système modulaire d'implant d'essai selon l'une des revendications précédentes, **caractérisé en ce que** le système modulaire d'implant d'essai (12) comprend au moins une deuxième partie d'implant d'essai (14, 16) ou au moins une partie d'implant et **en ce que** l'au moins une deuxième partie d'implant d'essai (14, 16) ou l'au moins une partie d'implant comprend un cône de liaison (24, 26) pouvant être inséré dans le logement conique (22),
dans lequel, en particulier,
a) le système modulaire d'implant d'essai (12) comprend au moins deux deuxièmes parties d'implants d'essai (14, 16) ou au moins deux parties d'implants avec des cônes de liaison (24, 26) de longueurs différentes
et/ou
b) l'au moins une deuxième partie d'implant d'essai (14, 16) est réalisée sous la forme d'une tige d'essai (100, 102) pouvant être insérée dans une cavité osseuse ou sous la forme d'un corps de râpe.

15. Système modulaire d'implant d'essai selon l'une quelconque des revendications précédentes, **caractérisé en ce que**
a) l'au moins une première partie d'implant d'essai (10) présente une tête d'articulation d'essai (30) sphérique ou en forme de sphère, la tête d'articulation d'essai (30) présentant en particulier une découpe d'une surface sphérique (32) à symétrie de rotation par rapport à l'axe longitudinal de l'implant (28),
dans lequel, en outre, en particulier, l'incision (48) relie le logement conique (22) à la surface sphérique (32) par une action de fluide,
et/ou
b) le système modulaire d'implant d'essai (12) comprend au moins une articulation artificielle d'essai (104, 106) avec une première partie articulaire d'implant d'essai (18, 20) et une deuxième partie d'implant d'articulation d'essai (108, 110) coopérant de manière articulée avec celle-ci,
dans lequel, en particulier, l'articulation artificielle d'essai (104, 106) étant réalisée sous la forme d'une articulation de hanche d'essai, la première partie articulaire d'implant d'essai (18, 20) formant une tige de prothèse avec une tête d'articulation d'essai et la deuxième partie d'implant d'articulation d'essai (108, 110) étant réalisée sous la forme d'une cupule articulaire d'essai (112, 114), et/ou
c) l'au moins une première partie d'implant d'essai (10) et/ou l'au moins une deuxième partie d'implant d'essai (14, 16) ou la partie d'implant étant réalisées d'une seule pièce, en particulier de manière monolithique.
